# EUROPÄISCHE PATENTSCHRIFT

(11) **EP 2 193 122 B1**
(45) Veröffentlichungstag und Bekanntmachung des Hinweises auf die Patenterteilung: **03.08.2016**
(21) Anmeldenummer: 08801940.1
(22) Anmeldetag: 09.09.2008
(51) Int. Cl.: C07D 233/94, C07D 401/12, C07D 403/10, C07D 403/12, C07D 405/06, C07D 405/12, C07D 405/14, C07D 409/06, C07D 409/12, C07D 417/12, A61K 31/4178

(54) **IMIDAZOLDERIVATE**
IMIDAZOLE DERIVATIVES
DÉRIVÉS D'IMIDAZOLE

(30) Priorität: 05.10.2007 DE 102007047738
(43) Veröffentlichungstag der Anmeldung: 09.06.2010
(73) Patentinhaber: Merck Patent GmbH, 64293 Darmstadt (DE)
(72) Erfinder: SCHULTZ, Melanie, 64287 Darmstadt (DE); SCHIEMANN, Kai, 64342 Seeheim-Jugenheim (DE); BOTTON, Gérard, F-78530 Buc (FR); BLAUKAT, Andree, 69198 Schriesheim (DE); KOBER, Ingo, 64521 Gross-Gerau (DE)
(86) Internationale Anmeldenummer: PCT/EP2008/007367
(87) Internationale Veröffentlichungsnummer: WO 2009/046804

(56) Entgegenhaltungen:
- EP-A- 1 258 484
- WO-A-2007/014619

## Beschreibung

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verbindungen zur Behandlung von Krankheiten, die mit einer Erhöhung des Lysophosphatsäure Spiegels einhergehen, ferner pharmazeutische Zusammensetzungen, die diese Verbindungen enthalten.

Im einzelnen betrifft die vorliegende Erfindung Verbindungen die bevorzugt eines oder mehrere Enzyme hemmen, die den Lysophosphatsäure (*lysophosphatidic acid* oder abgekürzt LPA) Spiegel regulieren und/oder modulieren, Zusammensetzungen, die diese Verbindungen enthalten, sowie Verfahren zu ihrer Verwendung zur Behandlung von Krankheiten und Leiden wie Angiogenese, Krebs, Tumorentstehung, -wachstum und -verbreitung, Arteriosklerose, Augenerkrankungen, choroidale Neovaskularisierung und diabetische Retinopathie, Entzündungserkrankungen, Arthritis, Neurodegeneration, Restenose, Wundheilung oder Transplantatabstossung. Insbesondere eignen sich die erfindungsgemäßen Verbindungen zur Therapie oder Prophylaxe von Krebserkrankungen.

Autotaxin (ATX) ist eine Enzym welches für die Erhöhung des Lysophosphatsäurespiegel in Ascites und Plasma verantwortlich ist (Xu et al. 1995, Clinical Cancer Research Vol. 1, Seite 1223 und Xu et al. 1995, Biochem. J. Vol- 309, Seite 933). ATX setzt Lysophatidylcholin (LPC) zu Lysophosphatsäure um (Tokumura et al. 2002, J. Biol. Chem., Vol 277, Seite 39436 und Umezu-Gozo et al. 2002, J. Biol. Chem., Vol. 158, Seite 227) LPA ist ein interzellularer Lipid Mediator der eine Vielzahl von biologischen und biochemischen Prozessen wie beispielsweise glatte Muskelkontraktion, Thrombozyten Aggregation und Apoptose beeinflusst (Tigyi et al. 2003 Prog. Lipid Res. Vol 42, Seite. 498 und Mills et al. 2003 Nat. Rev. Cancer Vol. 3, Seite 582 und Lynch et al. 2001 Prost. Lipid Med. Vol.64, Seite 33). Außerdem ist LPA in erhöhten Konzentrationen in Plasma und Ascites Flüssigkeit von Ovarial Krebs Patienten der frühen und späten Phase zu finden. LPA spielt dort eine Rolle bei der Tumorzellproliferation und deren Invasion in benachbarte Gewebe, welche zur Metastasierung führen kann (Xu et al. 1995, Clinical Cancer Research Vol. 1, Seite 1223 und Xu et al. 1995, Biochem. J. Vol- 309, Seite 933). Diese biologischen und phatobiologischen Prozesse werden durch die Aktivierung durch LPA von G-Protein gekoppelten Rezeptoren angeschaltet (Contos et al. 2000, Mol. Pharm. Vol 58, Seite. 1188).

Aus diesem Grunde ist es zur Behandlung von Tumor Patienten wünschenswert, den LPA Spiegel zu senken. Dies kann durch die Hemmung von Enzymen erreicht werden, die an der LPA Biosynthese beteiligt sind, wie beispielsweise Autotaxin (ATX, Sano et al. 2002, J. Biol. Chem. Vol. 277 , Seite 21197 und Aoki et al. 2003, J. Biol. Chem. Vol. 277 Seite 48737). Autotaxin gehört zu der Enzym Familie der Nukleotide Pyrophosphatasen und Phosphodiesterasen (Goding et al. 1998, Immünol. Rev. Vol. 161, Seite 11) und stellt einen wichtigen Ansatzpunkt bei der antitumoralen Therapie dar (Mills et al. 2003 Nat. Rev. Cancer Vol. 3, Seite 582 and Goto etal. 2004 J. Cell. Biochem. Vol. 92, Seite 1115), da es in Tumoren verstärkte expremiert wird und Tumorzellproliferation und - invasion in benachbarte Gewebe, was zur Metastasenbildung führen kann, bewirkt (Nam et al. 2000, Oncogene, Vol. 19 Seite 241). Außerdem bewirkt Autotaxin zusammen mit anderen angiogenetischen Faktoren Blutgefäßformation im Rahmen der Angiogenese (Nam et al. 2001, Cancer Res. Vol. 61 Seite. 6938). Angiogenese ist ein wichtiger Vorgang beim Tumorwachstum, der die Versorgung des Tumors mit Nährstoffen sichert. Aus diesem Grunde ist die Hemmung der Angiogenese ein wichtiger Ansatzpunkt der Krebs- und Tumortherapie, mit dem der Tumor gewissermaßen ausgehungert werden kann (Folkman, 2007, Nature Reviews Drug Discovery Vol. 6, Seite 273-286).

Es wurde überraschend gefunden, dass die erfindungsgemäßen Verbindungen eine spezifische Inhibierung der Enzymfamilie der Nukleotidepyrophosphatasen und Phosphodiesterasen, insbesondere Autotaxin bewirken. Die erfindungsgemäßen Verbindungen zeigen bevorzugt eine vorteilhafte biologische Aktivität, die in den, zum Beispiel hierin beschrieben Test, leicht nachweisbar ist. In derartigen Tests zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Generell können alle soliden und nicht soliden Tumore mit den Verbindungen der Formel I behandelt werden, wie z.B. die Monozytenleukämie, Hirn-, Urogenital-, Lymphsystem-, Magen-, Kehlkopf-Ovarial- und Lungenkarzinom, darunter Lungenadenokarzinom und kleinzelliges Lungenkarzinom. Zu weiteren Beispielen zählen Prostata-, Bauchspeicheldrüsen- und Brustkarzinom.

Wie hierin besprochen, sind Wirkungen der erfindungsgemäßen Verbindung für verschiedene Erkrankungen relevant. Dementsprechend sind die erfindungsgemäßen Verbindungen nützlich bei der Prophylaxe und/oder Behandlung von Erkrankungen, die durch eine Inhibierung einer oder mehrerer Nukleotidepyrophosphatasen und/oder Phosphodiesterasen, insbesondere Autotaxin, beeinflusst werden.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen.

Es kann gezeigt werden, dass die erfindungsgemäßen Verbindungen in einem Xenotranspiantat-Tumor-Modell eine vorteilhafte Wirkung aufweisen. Der Wirt oder Patient kann jeglicher Säugerspezies angehören, z. B. einer Primatenspezies, besonders Menschen; Nagetieren, einschließlich Mäusen, Ratten und Hamstern, Kaninchen, Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Sensitivität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Zellmigration zu inhibieren oder die zelluläre Sekretion von angiogenesefördernden Substanzen zu blockieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden können.

### STAND DER TECHNIK

Verbindungen, die zur Hemmung von Autotaxin fähig sind, sind in Peng et al. Bioorganic & Medicinal Chemistry Letters (17, 2007, Seite 1634-1640) beschrieben. Die dort beschriebenen Verbindungen stellen Lipid Analoga dar, welche strukturell keine Gemeinsamkeiten mit den erfindungsgemäßen Verbindungen aufweisen.
Andere lmidazol-carboxamide sind in FR 2889190 beschrieben.

### ZUSAMMENFASSUNG DER ERFINDUNG

Als nächster Stand der Technik gelten die Anmeldungen WO2007/014619 und EP 1 258 484. WO2007/014619 beschreibt Imidazolcarboxamid-Derivate zur Inhibierung von Fuctose- 1,6-bisphosphatase zur Behandlung von Patienten mit Insulinresistenzsyndrom. EP 1 258 484 beschreibt Isoxazol- und Thiazolverbindungen zur Verwendung als Lysophosphatidinsäure (LPA)-Rezeptorantagonist zur Behandlung von Patienten mit Zellproliferationserkrankungen wir Tumorerkrankungen. Entzündungserkrankungen, Nierenerkrankungen, Nervenstörungen und Aterienverstopfung.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter den genannten erfindungsgemäßen Verbindungen werden auch die Solvate verstanden.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüber hinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.
Die Bezeichnung "therapeutisch wirksame Menge" umfasst auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Die genannten erfinfungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so dass man sie aus dem Reaktionsgemisch nicht isoliert, sondern sofort weiter zu den Verbindungen der Formel I umsetzt.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist Pyridin, Acetonitril, Dichlormethan und/oder DMF.

Die Ausgangsverbindungen sind in der Regel bekannt. Sind sie neu, so können sie aber nach an sich bekannten Methoden hergestellt werden.
Die Edukte sind im Allgemeinen auch kommerziell erhältlich.

Die Reaktion gelingt vorzugsweise in Gegenwart eines Dehydratisierungsmittels, wie z.B. eines Carbodiimids wie N,N'-Dicyclohexylcarbodiimid ("DCCI"), 1,1'-Carbonyl-diimidazol oder N-3-Dimethylaminopropyl-N'-ethyl-carbodiimid ("DAPECI"), ferner Propanphosphonsäureanhydrid (vgl. Angew. Chem. 92, 129 (1980)), Diphenylphosphorylazid oder 2-Ethoxy-N-ethoxycarbonyl-1,2-dihydrochinolin.
Die Umsetzung erfolgt in der Regel in Gegenwart eines säurebindenden Mittels vorzugsweise einer organischen Base wie DIPEA, Triethylamin, Dimethylanilin, Pyridin oder Chinolin.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.
Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan öder Nitrobenzol; Ester wie Ethylacetat oder Gemische der genannten Lösungsmittel.
Besonders bevorzugt ist Acetonitril, Dichlormethan und/oder DMF.

Die genannten erfindungsgemäßen Verbindungen lassen sich in ihrer endgültigen Nichtsalzform verwenden. Andererseits umfasst die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen werden größtenteils konventionell hergestellt. Sofern die Verbindung eine Carbonsäuregruppe enthält, lässt sich eines ihrer geeigneten Salze dadurch bilden, dass man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und

N-Methylglutamin. Die Aluminiumsalze der Verbindungen der zählen ebenfalls dazu. Bei bestimmten Verbindungen lassen sich Säureadditionssalze dadurch bilden, dass man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, lodid, lsethionat, lsobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen werden dadurch hergestellt, dass man die freie Basenform mit einer ausreichenden Menge der gewünschten Säure in Kontakt bringt, wodurch man auf übliche Weise das Salz darstellt. Die freie Base lässt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.
Wie erwähnt werden die pharmazeutisch unbedenklichen Bäsenadditionssalze der genannten erfindungsgemäßen Verbindungen mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, dass man die freie Säureform mit einer ausreichenden Menge der gewünschten Base in Kontakt bringt, wodurch man das Salz auf übliche Weise darstellt. Die freie Säure lässt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfasst die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, dass unter dem Ausdruck "pharmazeutisch unbedenkliches Salz" im vorliegenden Zusammenhang ein Wirkstoff zu verstehen ist, der eine Verbindung in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine genannten erfiridungsgemäßen Verbindung und/oder ihre pharmazeutisch verwendbaren Salze und Stereoisomere, einschließlich deren Mischungen in allen Verhältnissen, sowie gegebenenfalls Träger- und/oder Hilfsstoffe.
Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepasste pharmazeutische Formulierungen können als separate Einheiten, wie z.B. Kapseln oder Tabletten; Pulver oder Granulate; Lösungen oder Suspensionen in wässrigen oder nichtwässrigen Flüssigkeiten; essbare Schäume oder Schaumspeisen; oder Öl-in-Wasser-Flüssigemulsionen oder Wasser-in-Öl-Flüssigemulsionen dargereicht werden.

So lässt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem essbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpresst wird, ein Schmiermittel und ein Sprengmittel zugegeben werden und das Ganze zu Tabletten verpresst wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch lässt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepresst wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengussformen zu verhindern. Das gefettete Gemisch wird dann zu Tabletten verpresst. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpresst werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so dass eine gegebene Quantität eine vorgegebene Menge der Verbindung enthält. Sirupe lassen sich herstellen, indem die Verbindung in einer wässrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung lässt sich auch so herstellen, dass die Freisetzung verlängert oder retardiert wird, wie beispielsweise durch Beschichtung oder Einbettung von partikulärem Material in Polymere, Wachs u.ä.

Die genannten erfindungsgemäßen Verbindungen sowie Salze und Solvate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.
Die genannten erfindungsgemäßen Verbindungen sowie die Salze und Solvate können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.
An die transdermale Verabreichung angepasste pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepasste pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepassten pharmazeutischen Formulierungen gehören Augentropfen, wobei der Wirkstoff in einem geeigneten Träger, insbesondere einem wässrigen Lösungsmittel, gelöst oder suspendiert ist.

An die topische Applikation im Mund angepasste pharmazeutische Formulierungen umfassen Lutschtabletten, Pastillen und Mundspülmittel.

An die rektale Verabreichung angepasste pharmazeutische Formulierungen können in Form von Zäpfchen oder Einläufen dargereicht werden.

An die nasale Verabreichung angepasste pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepasste pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepasste pharmazeutische Formulierungen können als Pessare, Tampons, Cremes, Gele, Pasten, Schäume oder Sprayformulierungen dargereicht werden.

Zu den an die parenterale Verabreichung angepassten pharmazeutischen Formulierungen gehören wässrige und nichtwässrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wässrige und nichtwässrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so dass nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, dass die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer der genannten erfindungsgemäßen Verbindung hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im Allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so dass die Gesamttagesdosis die gleiche ist. Eine wirksame Menge eines Salzes oder Solvats davon kann als Anteil der wirksamen Menge der erfindungsgemäßen Verbindung *per se* bestimmt werden. Es lässt sich annehmen, dass ähnliche Dosierungen für die Behandlung der anderen, obenerwähnten Krankheitszustände geeignet sind.
Gegenstand der Erfindung sind ferner Arzneimittel enthaltend mindestens eine genannten erfindungsgemäßen Verbindung und/oder ihre pharmazeutisch verwendbaren und Stereoisomere, einschließlich deren Mischungen in allen Verhältnissen, und mindestens einen weiteren Arzneimittelwirkstoff.

Bevorzugt aber nicht ausschliesslich werden die Arzneimittel der Tabelle 1 mit den eine genannten erfindungsgemäßen Verbindungen kombiniert. Eine Kombination genannten erfindungsgemäßen

| | | |
|---|---|---|
| Tabelle 1. | | |
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | |
| | Ormiplatin | BBR-3464 (Hoffrnann-La Roche) |
| | Iproplatin | |
| | | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexate | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder | Gimatecan (Sigma- Tau) |
| | Mitoxantron | Diflomotecan (Beaufour-Ipsen) |
| | Irinotecan (CPT-11) | |
| | 7-Ethyl-10-hydroxycamptothecin | TAS-103 (Taiho) |
| | | Elsamitrucin (Spectrum) |
| | Topotecan | J-107088 (Merck & Co) |
| | Dexrazoxanet (TopoTarget) | BNP-1350 (BioNumerik) |
| | | CKD-602 (Chong Kun Dang) |
| | Pixantron (Novuspharrna) | |
| | Rebeccamycin-Analogon (Exelixis) | KW-2170 (Kyowa Hakko) |
| | BBR-3576 (Novuspharrna) | |
| | | |
| Antitumor-Antibiotika | Dactinomycin (Actinomycin D) | Amonafid |
| | | Azonafid |
| | Doxorubicin (Adriamycin) | Anthrapyrazol |
| | Deoxyrubicin | Oxantrazol |
| | Valrubicin | Losoxantron |
| | Daunorubicin (Daunomycin) | Bleomycinsulfat (Blenoxan) |
| | Epirubicin | Bleomycinsäure |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem Pharmaceuticals) |
| | Cyanomorpholinodoxorubi cin | |
| | Mitoxantron (Novantron) | |
| | | |
| Antimitotische Mittel | Paclitaxel | SB 408075 (GlaxoSmithKline) |
| | Docetaxel | |
| | Colchicin | E7010 (Abbott) |
| | Vinblastin | PG-TXL (Cell Therapeutics) |
| | Vincristin | |
| | Vinorelbin | IDN 5109 (Bayer) |
| | Vindesin | A 105972 (Abbott) |
| | Dolastatin 10 (NCI) | A 204197 (Abbott) |
| | Rhizoxin (Fujisawa) | LU 223651 (BASF) |
| | Mivobulin (Warner-Lambert) | D 24851 (ASTA Medica) |
| | | ER-86526 (Eisai) |
| | Cemadotin (BASF) | Combretastatin A4 (BMS) |
| | RPR 109881A (Aventis) | Isohomohalichondrin-B (PharmaMar) |
| | TXD 258 (Aventis) | |
| | Epothilon B (Novartis) | ZD 6126 (AstraZeneca) |
| | T 900607 (Tularik) | PEG-Paclitaxel (Enzon) |
| | T 138067 (Tularik) | AZ10992 (Asahi) |
| | Cryptophycin 52 (Eli Lilly) | !DN-5109 (Indena) |
| | Vinflunin (Fabre) | AVLB (Prescient NeuroPharma) |
| | Auristatin PE (Teikoku Hormone) | |
| | | Azaepothilon B (BMS) |
| | BMS 247550 (BMS) | BNP- 7787 (BioNumerik) |
| | BMS 184476 (BMS) | CA-4-Prodrug (OXiGENE) |
| | BMS 188797 (BMS) | Dolastatin-10 (NrH) |
| | Taxoprexin (Protarga) | CA-4 (OXiGENE) |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylatsyntha se-Inhibitoren | Pemetrexed (Eli Lilly) | Nolätrexed (Eximias) |
| | ZD-9331 (BTG) | CoFactor™ (BioKeys) |
| | | |
| DNA-Antagonisten | Trabectedin (PharmaMar) | Mafosfamid (Baxter International) |
| | Glufosfamid (Baxter International) | |
| | | Apaziquon (Spectrum Pharmaceuticals) |
| | Albumin + 32P (Isotope Solutions) | |
| | | O6-Benzylguanin (Paligent) |
| | Thymectacin (NewBiotics) | |
| | Edotreotid (Novartis) | |
| | | |
| Farnesyltransfera se-Inhibitoren | Arglabin (NuOncology Labs) | Tipifarnib (Johnson & Johnson) |
| | Ionafarnib (Schering-Plough) | Perillylalkohol (DOR BioPharma) |
| | BAY-43-9006 (Bayer) | |
| | | |
| Pumpen-Inhibitoren | CBT-1 (CBA Pharma) | Zosuquidar-Trihydrochlorid (Eli Lilly) |
| | Tariquidar (Xenova) | |
| | (MS-209 (Schering AG) | Biricodar-Dicitrat (Vertex) |
| | | |
| Histonacetyltransf erase-Inhibitoren | Tacedinalin (Pfizer) | Pivaloyloxymethylbutyrat (Titan) |
| | SAHA (Aton Pharma) | |
| | MS-275 (Schering AG) | Depsipeptid (Fujisawa) |
| | | |
| Metalloproteinase-Inhibitoren Ribonucleosidred uktase-Inhibitoren | Neovastat (Aeterna Laboratories) | CMT -3 (CollaGenex) |
| | | BMS-275291 (Celltech) |
| | Marimastat (British Biotech) | Tezacitabin (Aventis) |
| | | Didox (Molecules for Health) |
| | Galliummaltolat (Titan) | |
| | Triapin (Vion) | |
| | | |
| TNF-alpha-Agonisten/Antago nisten | Virulizin (Lorus Therapeutics) | Revimid (Celgene) |
| | CDC-394 (Celgene) | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezepto r-Agonisten | Fenretinid (Johnson & Johnson) | Alitretinoin (Ligand) |
| | LGD-1550 (Ligand) | |
| | | |
| Immunmodulatore *n* | Interferon | Dexosom-Therapie (Anosys) |
| | Oncophage (Antigenics) | |
| | GMK (Progenics) | Pentrix (Australian Cancer Technology) |
| | Adenokarzinom-Impfstoff (Biomira) | |
| | | JSF-154 (Tragen) |
| | CTP-37 (AVI BioPharma)) | Krebsimpfstoff (Intercell) |
| | JRX-2 (Immuno-Rx) | Norelin (Biostar) |
| | PEP-005 (Peplin Biotech) | BLP-25 (Biomira) |
| | Synchrovax-Impfstoffe (CTL Immuno) | MGV (Progenics) |
| | | !3-Alethin (Dovetail) |
| | Melanom-Impfstoff (CTL Immuno) | CLL-Thera (Vasogen) |
| | p21-RAS-Impfstoff (GemVax) | |
| | | |
| Hormonelle und antihormonelle Mittel | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproa t | Goserelin |
| | | Leuporelin |
| | Medroxyprogesteron | Bicalutamid |
| | Testosteron | Flutamid |
| | Testosteronpropionat | Octreotid |
| | Fluoxymesteron | Nilutamid |
| | Methyltestosteron | Mitotan |
| | Diethylstilbestrol | P-04 (Novogen) |
| | Megestrol | 2-Methoxyöstradiol (EntreMed) |
| | Tamoxifen | |
| | Toremofin | Arzoxifen (Eli Lilly) |
| | Dexamethason | |
| | | |
| Photodynamische Mittel | Talaporfin (Light Sciences) | Pd-Bacteriopheophorbid (Yeda) |
| | Theralux (Theratechnotogies) | |
| | | Lutetium-Texaphyrin (Pharmacyclics) |
| | Motexafin-Gadolinium (Pharmacyclics) | |
| | | Hypericin |
| | | |
| Tyrosinkinase-Inhibitoren | Imatinib (Novartis) | Kahalid F (PharmaMar) |
| | Leflunomid (Sugen/Pharmacia) | CEP- 701 (Cephalon) |
| | | CEP-751 (Cephalon) |
| | ZDI839 (AstraZeneca) | MLN518 (Millenium) |
| | Erlotinib (Oncogene Science) | PKC412 (Novartis) |
| | | Phenoxodiol O |
| | Canertjnib (Pfizer) | Trastuzumab (Genentech) |
| | Squalamin (Genaera) | C225 (ImClone) |
| | SU5416 (Pharmacia) | rhu-Mab (Genentech) |
| | SU6668 (Pharmacia) | MDX-H210 (Medarex) |
| | ZD4190 (AstraZeneca) | 2C4 (Genentech) |
| | ZD6474 (AstraZeneca) | MDX-447 (Medarex) |
| | Vatalanib (Novartis) | ABX-EGF (Abgenix) |
| | PKI166 (Novartis) | IMC-1C11 (ImClone) |
| | GW2016 (GlaxoSmithKline) | |
| | EKB-509 (Wyeth) | |
| | EKB-569 (Wyeth) | |
| Verschiedene Mittel | SR-27897 (CCK-A-Inhibitor, Sanofi-Synthelabo) | BCX-1777 (PNP-Inhibitor, BioCryst) |
| | | Ranpirnase (Ribonuclease-Stimulans, Alfacell) |
| | Tocladesin (cyclisches-AMP-Agonist, Ribapharm) | |
| | Alvocidib (CDK-Inhibitor, Aventis) | Galarubicin (RNA-Synthese-Inhibitor, Dong-A) |
| | CV-247 (COX-2-Inhibitor, Ivy Medical) | |
| | | Tirapazamin (Reduktionsmittel, SRI International) |
| | P54 (COX-2-Inhibitor, Phytopharm) | |
| | CapCell™ (CYP450- | N-Acetylcystein |
| | Stimulans, Bavarian Nordic) | (Reduktionsmittel, Zambon) |
| | GCS-IOO (gal3-Antagonist, GlycoGenesys) | R-Flurbiprofen (NF-kappaB-Inhibitor, Encore) |
| | | 3CPA (NF-kappaB-Inhibitor, Active Biotech) |
| | G17DT-Immunogen (Gastrin-Inhibitor, Aphton) | |
| | | Seocalcitol (Vitamin-D-Rezeptor-Agonist, Leo) |
| | Efaproxiral (Oxygenator, Allos Therapeutics) | |
| | | 131-I-TM-601 (DNA-Antagonist, TransMolecular) |
| | PI-88 (Heparanase-Inhibitor, Progen) | |
| | Tesmilifen (Histamin-Antagonist, YM BioSciences) | Eflornithin (ODC-Inhibitor, ILEX Oncology) |
| | | Minodronsäure (Osteoclasten-Inhibitor, Yamanouchi) |
| | Histamin (Histamin-H2-Rezeptor- Agonist, Maxim) | |
| | Tiazofurin (IMPDH-Inhibitor, Ribapharm) | Indisulam (p53-Stimulans, Eisai) |
| | Cilengitid (Integrin-Antagonist, Merck KGaA) | Aplidin (PPT-Inhibitor, PharmaMar) |
| | SR-31747 (IL-1-Antagonist, Sanofi-Synthelabo) | Rituximab (CD20-Antikörper, Genentech) |
| | | Gemtuzumab (CD33-Antikörper, Wyeth Ayerst) |
| | CCI-779 (mTOR-Kinase-Inhibitor, Wyeth) | |
| | Exisulind (PDE-V-Inhibitor, Cell Pathways) | PG2 (Hämatopoese-Verstärker, Pharmagenesis) |
| | CP-461 (PDE-V-Inhibitor, Cell Pathways) | Immunol™ (Triclosan-Oralspülung, Endo) |
| | AG-2037 (GART-Inhibitor, Pfizer) | Triacetyluridin (Uridin-Prodrug, Wellstat) |
| | WX-UK1 (Plasminogenaktivator-Inhibitor, Wilex) | SN-4071 (Sarkom-Mittel, Signature BioScience) |
| | | TransMID-107™ (Immunotoxin, KS Biomedix) |
| | PBI-1402 (PMN-Stimulans, ProMetic LifeSciences) | |
| | Bortezomib (Proteasom-Inhibitor, Millennium) | PCK-3145 (Apoptose-Förderer, Procyon) |
| | SRL-172 (T-Zell-Stimulans, SR Pharma) | Doranidazol (Apoptose-Förderer, Pola) |
| | TLK-286 (Glutathion-S-Transferase-Inhibitor, Telik) | CHS-828 (cytotoxisches Mittel, Leo) |
| | | trans-Retinsäure (Differentiator, NIH) |
| | PT-100 (Wachstumsfaktor-Agonist, Point Therapeutics) | |
| | | MX6 (Apoptose-Förderer, MAXIA) |
| | Midostaurin (PKC-Inhibitor, | Apomin (Apoptose- |
| | Novartis) | Förderer, ILEX Oncology) |
| | Bryostatin-1 (PKC-Stimulans, GPC Biotech) | Urocidin (Apoptose-Förderer, Bioniche) |
| | CDA-II (Apoptose-Förderer, Everlife) | Ro-31-7453 (Apoptose-Förderer, La Roche) |
| | SDX-101 (Apoptose-Förderer, Salmedix) | Brostallicin (Apoptose-Förderer, Pharmacia) |
| | Ceflatonin (Apoptose-Förderer, ChemGenex) | |
| | | |
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | |
| | Ormiplatin | BBR-3464 (Hoffrnann-La Roche) |
| | Iproplatin | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexate | Ethinylcytidin (Taiho) |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder | Gimatecan (Sigma- Tau) |
| | Mitoxantron | Diflomotecan (Beaufour-Ipsen) |
| | Irinotecan (CPT-11) | |
| | 7-Ethyl-10-hydroxycamptothecin | TAS-103 (Taiho) |
| | | Elsamitrucin (Spectrum) |
| | Topotecan | J-107088 (Merck & Co) |
| | Dexrazoxanet (TopoTarget) | BNP-1350 (BioNumerik) |
| | | CKD-602 (Chong Kun Dang) |
| | Pixantron (Novuspharrna) | |
| | Rebeccamycin-Analogon (Exelixis) | KW-2170 (Kyowa Hakko) |
| | BBR-3576 (Novuspharrna) | |
| | | |
| Antitumor-Antibiotika | Dactinomycin (Actinomycin D) | Amonafid |
| | | Azonafid |
| | Doxorubicin (Adriamycin) | Anthrapyrazol |
| | Deoxyrubicin | Oxantrazol |
| | Valrubicin | Losoxantron |
| | Daunorubicin (Daunomycin) | Bleomycinsulfat (Blenoxan) |
| | Epirubicin | Bleomycinsäure |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem Pharmaceuticals) |
| | Cyanomorpholinodoxorubi cin | |
| | Mitoxantron (Novantron) | |
| Antimitotische Mittel | Paclitaxel | SB 408075 (GlaxoSmithKline) |
| | Docetaxel | |
| | Colchicin | E7010 (Abbott) |
| | Vinblastin | PG-TXL (Cell Therapeutics) |
| | Vincristin | |
| | Vinorelbin | IDN 5109 (Bayer) |
| | Vindesin | A 105972 (Abbott) |
| | Dolastatin 10 (NCI) | A 204197 (Abbott) |
| | Rhizoxin (Fujisawa) | LU 223651 (BASF) |
| | Mivobulin (Warner-Lambert) | D 24851 (ASTA Medica) |
| | | ER-86526 (Eisai) |
| | Cemadotin (BASF) | Combretastätin A4 (BMS) |
| | RPR 109881A (Aventis) | Isohomohalichondrin-B (PharmaMar) |
| | TXD 258 (Aventis) | |
| | Epothilon B (Novartis) | ZD 6126 (AstraZeneca) |
| | T 900607 (Tularik) | PEG-Paclistaxel (Enzon) |
| | T 138067 (Tularik) | AZ10992 (Asahi) |
| | Cryptophycin 52 (Eli Lilly) | !DN-5109 (Indena) |
| | Vinflunin (Fabre) | AVLB (Prescient NeuroPharma) |
| | Auristatin PE (Teikoku Hormone) | |
| | | Azaepothilon B (BMS) |
| | BMS 247550 (BMS) | BNP- 7787 (BioNumerik) |
| | BMS 184476 (BMS) | CA-4-Prodrug (OXiGENE) |
| | BMS 188797 (BMS) | Dolastatin-10 (NrH) |
| | Taxoprexin (Protarga) | CA-4 (OXiGENE) |
| | | |
| Aromatase-Inhibitoren | Aminoglutethimid | Exemestan |
| | Letrozol | Atamestan (BioMedicines) |
| | Anastrazot | YM-511 (Yamanouchi) |
| | Formestan | |
| | | |
| Thymidylatsyntha se-Inhibitoren | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter International) |
| | Glufosfamid (Baxter International) | |
| | | Apaziquon (Spectrum Pharmaceuticals) |
| | Albumin + 32P (Isotope Solutions) | |
| | | O6-Benzylguanin (Paligent) |
| | Thymectacin (NewBiotics) | |
| | Edotreotid (Novartis) | |
| | | |
| Farnesyltransfera se-Inhibitoren | Arglabin (NuOncology Labs) | Tipifarnib (Johnson & Johnson) |
| | Ionafarnib (Schering-Plough) | Perillylalkohol (DOR BioPharma) |
| | BAY-43-9006 (Bayer) | |
| Pumpen-Inhibitoren | CBT-1 (CBA Pharma) | Zosuquidar-Trihydrochlorid (Eli Lilly) |
| | Tariquidar (Xenova) | |
| | MS-209 (Schering AG) | Biricodar-Dicitrat (Vertex) |
| | | |
| Histonacetyltransf erase-Inhibitoren | Tacedinalin (Pfizer) | Pivaloyloxymethylbutyrat (Titan) |
| | SAHA (Aton Pharma) | |
| | MS-275 (Schering AG) | Depsipeptid (Fujisawa) |
| | | |
| Metalloproteinase-Inhibitoren Ribonucleosidred uktase-Inhibitoren | Neovastat (Aeterna Laboratories) | CMT -3 (CollaGenex) |
| | | BMS-275291 (Celltech) |
| | Marimastat (British Biotech) | Tezacitabin (Aventis) |
| | | Didox (Molecules for Health) |
| | Galliummaltolat (Titan) | |
| | Triapin (Vion) | |
| | | |
| TNF-alpha-Agonisten/Antago nisten | Virulizin (Lorus Therapeutics) | Revimid (Celgene) |
| | CDC-394 (Celgene) | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezepto r-Agonisten | Fenretinid (Johnson & Johnson) | Alitretinoin (Ligand) |
| | LGD-1550 (Ligand) | |
| | | |
| Immunmodulatore n | Interferon | Dexosom-Therapie (Anosys) |
| | Oncophage (Antigenics) | |
| | GMK (Progenics) | Pentrix (Australian Cancer Technology) |
| | Adenokarzinom-Impfstoff (Biomira) | |
| | | JSF-154 (Tragen) |
| | CTP-37 (AVI BioPharma) | Krebsimpfstoff (Intercell) |
| | JRX-2 (Immuno-Rx) | Norelin (Biostar) |
| | PEP-005 (Peplin Biotech) | BLP-25 (Biomira) |
| | Synchrovax-Impfstoffe (CTL lmmuno) | MGV (Progenics) |
| | | !3-Alethin (Dovetail) |
| | Melanom-Impfstoff (CTL Immuno) | CLL-Thera (Vasogen) |
| | p21-RAS-Impfstoff (GemVax) | |
| Hormonelle und | Östrogene | Prednison |
| antihormonelle | konjugierte Östrogene | Methylprednisolon |
| Mittel | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol |
| | Toremofin | (EntreMed) |
| | Dexamethason | Arzoxifen (Eli Lilly) |
| | | |
| Photodynamische | Talaporfin (Light Sciences) | Pd-Bacteriopheophorbid (Yeda) |
| Mittel | Theralux (Theratechnologies) | Lutetium-Texaphyrin (Pharmacyclics) |
| | Motexafin-Gadolinium (Pharmacyclics) | Hypericin |
| | | |
| Tyrosinkinase-Inhibitoren | Imatinib (Novartis) | Kahalid F (PharmaMar) |
| | Leflunomid (Sugen/Pharmacia) | CEP- 701 (Cephalon) |
| | | CEP-751 (Cephalon) |
| | ZDI839 (AstraZeneca) | MLN518 (Millenium) |
| | Erlotinib (Oncogene Science) | PKC412 (Novartis) |
| | Canertjnib (Pfizer) | Phenoxodiol O |
| | Squalamin (Genaera) | Trastuzumab (Genentech) |
| | SU5416 (Pharmacia) | C225 (ImClone) |
| | SU6668 (Pharmacia) | rhu-Mab (Genentech) |
| | ZD4190 (AstraZeneca) | MDX-H210 (Medarex) |
| | ZD6474 (AstraZeneca) | 2C4 (Genentech) |
| | Vatalanib (Novartis) | MDX-447 (Medarex) |
| | PKI166 (Novartis) | ABX-EGF (Abgenix) |
| | GW2016 (GlaxoSmithKline) | IMC-1C11 (ImClone) |
| | EKB-509 (Wyeth) | |
| | EKB-569 (Wyeth) | |
| | | |
| Verschiedene Mittel | SR-27897 (CCK-A-Inhibitor, Sanofi-Synthelabo) | BCX-1777 (PNP-Inhibitor, BioCryst) |
| | Tocladesin (cyclisches- | Ranpirnase (Ribonuclease-Stimulans, Alfacell) |
| | AMP-Agonist, Ribapharm) | Galarubicin (RNA-Synthese-Inhibitor, Dong-A) |
| | Alvocidib (CDK-Inhibitor, Aventis) | |
| | | Tirapazamin (Reduktionsmittel, SRI International) |
| | CV-247 (COX-2-Inhibitor, Ivy Medical) | |
| | P54 (COX-2-Inhibitor, Phytopharm) | N-Acetylcystein (Reduktionsmittel, Zambon) |
| | CapCell™ (CYP450-Stimulans, Bavarian Nordic) | R-Flurbiprofen (NF-kappaB-Inhibitor, Encore) |
| | | 3CPA (NF-kappaB-Inhibitor, Active Biotech) |
| | GCS-IOO (gal3-Antagonist, GlycoGenesys) | |
| | | Seocalcitol (Vitamin-D-Rezeptor-Agonist, Leo) |
| | G17DT-Immunogen (Gastrin-Inhibitor, Aphton) | 131-I-TM-601 (DNA-Antagonist, TransMolecular) |
| | Efaproxiral (Oxygenator, Allos Therapeutics) | |
| | | Eflornithin (ODC-Inhibitor, ILEX Oncology) |
| | PI-88 (Heparanase-Inhibitor, Progen) | |
| | | Minodronsäure |
| | Tesmilifen (Histamin-Antagonist, YM BioSciences) | (Osteoclasten-Inhibitor, Yamanouchi) |
| | | Indisulam (p53-Stimulans, Eisai) |
| | Histamin (Histamin-H2-Rezeptor- Agonist, Maxim) | |
| | | Aplidin (PPT-Inhibitor, PharmaMar) |
| | Tiazofurin (IMPDH-Inhibitor, Ribapharm) | Rituximab (CD20-Antikörper, Genentech) |
| | Cilengitid (Integrin-Antagonist, Merck KGaA) | Gemtuzumab (CD33-Antikörper, Wyeth Ayerst) |
| | SR-31747 (IL-1-Antagonist, Sanofi-Synthelabo) | PG2 (Hämatopoese-Verstärker, Pharmagenesis) |
| | CCI-779 (mTOR-Kinase-Inhibitor, Wyeth) | Immunol™ (Triclosan-Oralspülung, Endo) |
| | Exisulind (PDE-V-Inhibitor, Cell Pathways) | Triacetyluridin (Uridin-Prodrug, Wellstat) |
| | CP-461 (PDE-V-Inhibitor, Cell Pathways) | SN-4071 (Sarkom-Mittel, Signature BioScience) |
| | AG-2037 (GART-Inhibitor, Pfizer) | TransMID-107™ (Immunotoxin, KS Biomedix) |
| | WX-UK1 (Plasminogenaktivator-Inhibitor, Wilex) | |
| | | PCK-3145 (Apoptose-Förderer, Procyon) |
| | PBI-1402 (PMN-Stimulans, ProMetic LifeSciences) | Doranidazol (Apoptose-Förderer, Pola) |
| | | CHS-828 (cytotoxisches Mittel, Leo) |
| | Bortezomib (Proteasom- | |
| | Inhibitor, Millennium) | trans-Retinsäure (Differentiator, NIH) |
| | SRL-172 (T-Zell-Stimulans, SR Pharma) | |
| | | MX6 (Apoptose-Förderer, MAXIA) |
| | TLK-286 (Glutathion-S-Transferase-Inhibitor, Telik) | |
| | | Apomin (Apoptose-Förderer, ILEX Oncology) |
| | PT-100 (Wachstumsfaktor-Agonist, Point Therapeutics) | Urocidin (Apoptose-Förderer, Bioniche) |
| | | Ro-31-7453 (Apoptose-Förderer, La Roche) |
| | Midostaurin (PKC-Inhibitor, Novartis) | Brostallicin (Apoptose-Förderer, Pharmacia) |
| | Bryostatin-1 (PKC-Stimulans, GPC Biotech) | |
| | CDA-II (Apoptose-Förderer, Everlife) | |
| | SDX-101 (Apoptose-Förderer, Salmedix) | |
| | Ceflatonin (Apoptose-Förderer, ChemGenex) | |

Bevorzugt werden die genannten erfindungsgemäßen Verbindungen mit den mit bekannten Antikrebsmitteln kombiniert: Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. Die synergistischen Wirkungen der Hemmung des VEGF in Kombination mit Radiotherapie sind in der Fachwelt beschrieben worden (siehe WO 00/61186). "Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1- piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Hemmer, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, lrofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis-[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyldaunorubicin (siehe WO 00/50032), was jedoch keine Einschränkung darstellen soll.
Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Hemmer sind zum Beispiel Topotecan, Hycaptamin, Irinotecan, Rubitecan, 6-Ethoxypropionyl-3',4'-O-exo-benzylidenchartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.

Zu den "antiproliferativen Mitteln" zählen Antisense-RNA- und-DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo-(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehydthiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

Insbesondere bevorzugt sind die genannten erfindungsgemäßen Verbindungen zür Behandlung und Prophylaxe von Tumorerkrankungen.

Der Tumor ist vorzugsweise ausgewählt aus der Gruppe der Tumoren des -Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge.

Der Tumor ist weiterhin vorzugsweise ausgewählt aus der Gruppe Lungenadenokarzinom, kleinzellige Lungenkarzinome, Bauchspeicheldrüsenkrebs, Ovarialkarzinom, Glioblastome, Kolonkarzinom und Brustkarzinom.

Weiterhin bevorzugt sind die genannten erfindungsgemäßen Verbindungen zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Unter einem anderen Aspekt umfasst die Erfindung ein zur Behandlung eines Patienten, der ein Neoplasma, wie einen Krebs, hat, durch Verabreichung einer Verbindung der Formel (I) in Kombination mit einem antiproliferativen Mittel. Geeignete antiproliferative Mittel umfassen die in Tabelle 1 bereitgestellten.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetet oder Dichlormethan, trennt ab, trocknet die organische Phase über Natriumsulfat, dampft ein und reinigt duch Chromatographie an Kieselgel und/oder durch Kristallisation. Rt-Werte werden per HPLC mit erwähnten Laufmitteln bestimmt.
Massenspektrometrie (MS): EI (Electronenstoß-Ionisation) M⁺
   FAB (Fast Atom Bombardment) (M+H)⁺
   ESI (Electrospray Ionization) (M+H)⁺
APCI-MS (atmospheric pressure chemical ionization - mass spectrometry) (M+H)⁺

### Methode LC/MS:

Solvent A: Wasser + 0,1 % TFA
Solvent B: Acetonitril + 0,1 % TFA
Flow: 2,4 ml/min
Gradient: 0,0 min 4 % B
   2,6 min 100 % B
Säule: Chromolith^{®} Speed ROD RP-18e 50-4, 6 mm

### Methode HPLC:

Solvent A: Wasser + 0,1 % TFA
Solvent B: Acetonitril + 0,08 % TFA
Flow: 1,5 ml/min
Gradient: 0,0 - 0,5 min 100 % A
   0,5 - 3,5 min auf 100 % B
   3,5 - 4,5 min 100 % B
   4,5 - 4,6 min auf 100 % A
   4,6 - 5,0 min 1000 % A
Säule: Si-ROD^{®} UM9423/100, 3 mm

### Beispiel 1

Die Synthese von (4-{4-Carbamoyl-5-[3-(4-isopropyl-phenyl)-ureido]-1H-imidazol-2-ylmethyl}-phenoxy)-essigsäure ("A1") erfolgt analog nachstehendem Schema 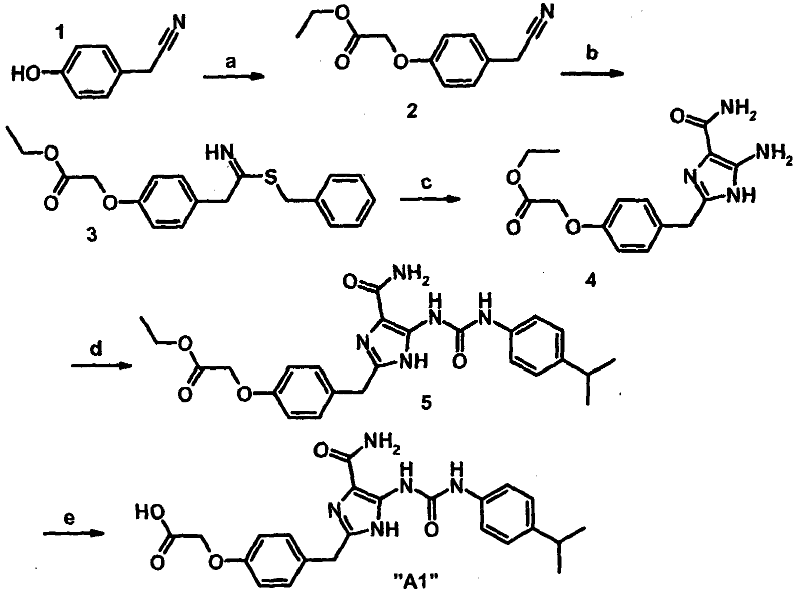
a. Verbindung 1 (13 g, 97.6 mmol) wird in Acetonitril (150 mL) vorgelegt, Kaliumcarbonat (16.2 g, 117 mmol), Natriumiodid (1.5 g, 9.8 mmol) und Bromessigsäureethylester (11.9 ml, 107.4 mmol) werden bei RT zugefügt. Die bräunliche Suspension wird für 6 h am Rückfluss gekocht und dann bei RT 18 h weitergerührt. Es entsteht eine gelbliche Lösung mit farblosem Niederschlag. Die Reaktionsmischung wird mit Wasser versetzt, wobei sich der Niederschlag löst, und die wässrige Phase 2 mal mit DCM extrahiert. Die vereinigten organischen Phasen werden 2 mal mit Wasser gewaschen, über Magnesiumsulfat getrocknet und eingeengt. Nach Trocknen des entstandenen Niederschlags erhält man 21.2 g (96.7 mmol, 99%) der Verbindung 2, die ohne weitere Aufreinigung umgesetzt wird.
b. Verbindung 2 (3.7 g, 17 mmol) und 2 ml Benzylmercaptan (17 mmol) werden in 50 ml absolutem Ether vorgelegt und auf 10 °C gekühlt. Bei dieser Temperatur leitet man über 30 Minuten vorsichtig Chlorwasserstoffgas durch die Lösung. Der Ansatz wird auf RT erwärmt und 16 h weitergerührt. Dann filtriert man den entstandenen Niederschlag ab und wäscht mit Diethylether. So isoliert man 5.8 g (3.5 mmol, 21%) des gewünschten Produktes 3 als farblosen Feststoff.
c. Verbindung 3 (1 g, 2.6 mmol), 2-Cyan-acetamid (261 mg, 2.6 mmol) und Natriumhydrogencarbonat (221 mg, 2.6 mmol) werden in einem Mikrowellengefäß in 20 ml absolutem THF gemischt, das Gefäß wird verschlossen und in einem Mikrowellengerät für 10 Min auf 100 °C erhitzt. Es entsteht ein farbloser Feststoff, den man absitzen lässt. Die überstehende Lösung wird vorsichtig abgenommen, der Niederschlag in neuem absolutem THF aufgeschlämmt, wieder absitzen lassen und die überstehende Lösung entfernt. Dieser Vorgang wird ein weiteres Mal wiederholt. Der Niederschlag wird erst an der Luft und dann im Vakuum getrocknet. Man erhält 702 mg (1.8 mmol, 69 %) der Verbindung 4 als farblosen Feststoff.
d. Verbindung 4 (200 mg, 0.6 mmol) wird in Pyridin (5 ml) gelöst. Nun werden 129 µL (0.8 mmol) p-Isopropyl Isocyanat zugegeben und die Reaktionsmischung 16 h bei RT gerührt. Man engt am Rotationsverdampfer ein und erhält aus dem braunen Rückstand mithilfe der präparativen HPLC die aufgereinigte Verbindung 5 als braunen Feststoff (41 mg, 0.07 mmol, 12 %).
e. Verbindung 5 (150 mg, 0.3 mmol) wird in einem Mikrowellengefäß in 4 ml Dioxan gelöst und mit 0.6 ml 1 N NaOH versetzt. Man erhitzt für 10 Min in der Mikrowelle auf 100°C. Man lässt auf RT abkühlen, säuert mit Salzsäure an, verteilt das Rohprodukt zwischen Wasser und Essigester. Die wässrige Phase wird zweimal mit Essigester extrahiert und die vereinigten organischen Phasen dann mit gesättigter NatriumchloridLösung gewaschen, über Magnesiumsulfat getrocknet und eingeengt. Das so erhaltene Rohprodukt wird an der präparativen HPLC weiter aufgereinigt. Man erhält Produkt "A1" (6 mg, 4%) als farblosen Feststoff; [M+H⁺] 452.19; Rt HPLC 2.61 [min].

Analog werden die nachstehenden genannten erfindungsgemäßen Verbindungen erhalten

| Verbindung Nr. | Name und/oder Struktur | ESI [M+H]⁺ | HPLC (RT in min) Methode |
|---|---|---|---|
| "A2" | 4-{4-Carbamoyl-5-[3-(4-isopropyl-phenyl)-ureido]-1H-imidazol-2-ylmethyl}-benzoesäure | 422.18 | 2.61 |
| | 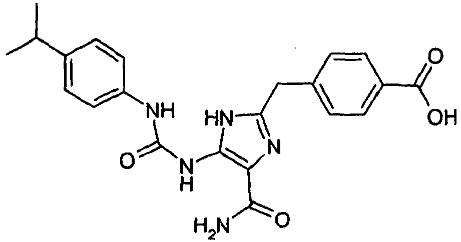 | | |
| "A3" | 4-{5-[3-(4-Butyl-phenyl)-ureido]-4-carbamoyl-1H-imidazol-2-ylmethyl}-benzoesäure | 436.19 | 2.73 |
| ¹H-NMR (MeOD): δ [ppm] = 0.95 (m, 3H), 1.36 (m, 2H). 1.58 (m, 2H), 2.58 (m, 2H), 4.16 (2, 2H), 7.12 (bd, J = 7 Hz, 2H), 7.35 (bd, J = 7 Hz, 2H), 7.41 (bd, J = 7Hz, 2 H), 8.01 (bd, J = 7Hz, 2H) | | | |
| "A4" | 4-[4-Carbamoyl-5-(3-hexyl-ureido)-1 H-imidazol-2-ylmethyl]-benzoesäure | 388.19 | 2.47 |
| "A5" | 4-[4-Carbamoyl-5-(3-p-tolyl-ureido)-1H-imidazol-2-ylmethyl]-benzoesäure | 394.14 | 2.41 |
| "A6" | 4-{4-Carbamoyl-5-[3-(4-chlor-phenyl)-ureido]-1H-imidazol-2-ylmethyl}-benzoesäure | 414.09 | 2.49 |
| "A7" | 4-{4-Carbamoyl-5-[3-(4-ethyl-phenyl)-ureido]-1H-imidazol-2-ylmethyl}-benzoesäure | 408.16 | 2.53 |
| "A8" | 4-{4-Carbamoyl-5-[3-(3-chlor-2-methyl-phenyl)-ureido]-1H-imidazol-2-ylmethyl}-benzoesäure | 428.1 | 2.65 |
| "A9" | 4-{5-[3-(4-Butoxy-phenyl)-ureido]-4-carbamoyl-1H-imidazol-2-ylmethyl}-benzoesäure | 452.19 | 2.65 |
| "A10" | 5-{4-Carbamoyl-5-[3-(4-trifluormethyl-phenyl)-ureido]-1H-imidazol-2-ylmethyl}-furan-2-carbonsäure | 438.09 | 2.57 |
| | 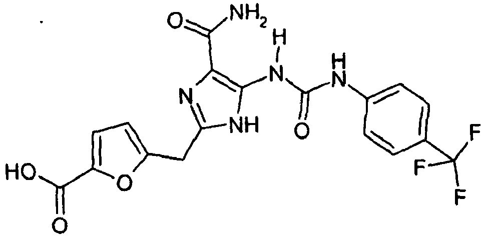 | | |
| | 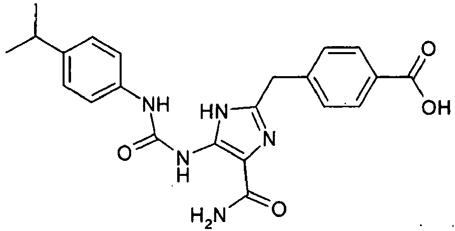 | | |
| "A6" | 4-{4-Carbamoyl-5-[3-(4-chlor-phenyl)-ureido]-1H-imidazol-2-ylmethyl}-benzoesäure | 414.09 | 2.49 |
| "A7" | 4-{4-Carbamoyl-5-[3-(4-ethyl-phenyl)-ureido]-1H-imidazol-2-ylmethyl}-benzoesäure | 408.16 | 2.53 |
| "A8" | 4-{4-Carbamoyl-5-[3-(3-chlor-2-methyl-phenyl)-ureido]-1H-imidazol-2-ylmethyl}-benzoesäure | 428.1 | 2.65 |
| "A9" | 4-{5-[3-(4-Butoxy-phenyl)-ureido]-4-carbamoyl-1H-imidazol-2-ylmethyl}-benzoesäure | 452.19 | 2.65 |
| "A10" | 5-{4-Carbamoyl-5-[3-(4-trifluormethyl-phenyl)-ureido]-1H-imidazol-2-ylmethyl}-furan-2-carbonsäure | 438.09 | 2.57 |
| | 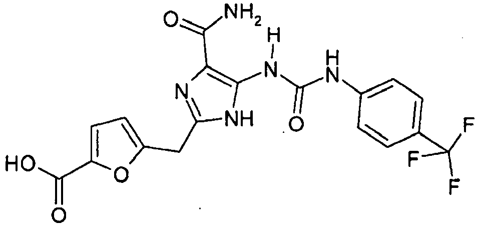 | | |
| "A11" | 5-{4-Carbamoyl-5-[3-(4-ethyl-phenyl)-ureido]-1H-imidazol-2-ylmethyl}-furan-2-carbonsäure | 398.14 | 2.51 |
| "A12" | 5-{4-Carbamoyl-5-[3-(4-isopropyl-phenyl)-ureido]-1H-imidazol-2-ylmethyl}-furan-2-carbonsäure | 412.15 | 2.6 |
| "A15" | 4-{4-Carbamoyl-5-[3-(4-trifluormethyl-phenyl)-ureido]-1H-imidazol-2-ylmethyl}-benzoesäure | 448.12 | 2.61 |
| ¹H-NMR (DMSO-d₆): δ [ppm] = 4.10 (bs, 2H), 7.07 (bm, 2H), 7.36 (bs, 2H), 7.64 (bs, 4H), 7.88 (bm, 2H), 9.06 (bs, 1H), 9.69 (bs, 1H), 11.45 (bs, 1H), 12.38 (bs, 1H) | | | |
| "A30" | 5-{4-Carbamoyl-5-[3-(4-chlor-phenyl)-ureido]-1H-imidazol-2-ylmethyl}-furan-2-carbonsäure | 404.07 | 2.46 |
| "A31" | 5-{4-Carbamoyl-5-[3-(2,4,6-trimethyl-phenyl)-ureido]-1H-imidazol-2-ylmethyl}-furan-2-carbonsäure | 412.15 | 2.27 |
| "A34" | 5-{4-Carbamoyl-5-[3-(2-fluor-5-trifluormethylphenyl)-ureido]-1H-imidazol-2-ylmethyl}-furan-2-carbonsäure | 456.09 | 2.57 |
| "A35" | 5-{4-Carbamoyl-5-[3-(4-chlor-2-trifluormethylphenyl)-ureido]-1H-imidazol-2-ylmethyl}-furan-2-carbonsäure | 472.06 | 2.61 |
| "A38" | (4-{4-Carbamoyl-5-[3-(4-trifluormethyl-phenyl)-ureido]-1H-imidazol-2-ylmethyl}-phenoxy)-essigsäure | 478.13 | 2.57 |
| "A39" | (4-{5-[3-(4-Butyl-phenyl)-ureido]-4-carbamoyl-1 H-imidazol-2-ylmethyl}-phenoxy)-essigsäure | 466.2 | 2.71 |
| "A40" | {4-[4-Carbamoyl-5-(3-p-tolyl-ureido)-1 H-imidazol-2-ylmethyl]-phenoxy}-essigsäure | 424.15 | 2.39 |
| "A41" | (4-{4-Carbamoyl-5-[3-(4-chlor-phenyl)-ureido]-1 H-imidazol-2-ylmethyl}-phenoxy)-essigsäure | 444.1 | 2.47 |
| "A42" | (4-{4-Carbamoyl-5-[3-(4-ethyl-phenyl)-ureido]-1H-imidazol-2-ylmethyl}-phenoxy)-essigsäure | 438.17 | 2.51 |
| "A43" | (4-{4-Carbamoyl-5-[3-(4-pentyl-phenyl)-ureido]-1H-imidazol-2-ylmethyl}-phenoxy)-essigsäure | 480.22 | 2.8 |
| "A49" | (4-{4-Carbamoyl-5-[3-(3-trifluormethyl-phenyl)-ureido]-1H-imidazol-2-yl}-phenoxy)-essigsäure | 464.11 | 2.66 |
| "A50" | (4-{4-Carbamoyl-5-[3-(4-trifluormethyl-phenyl)-ureido]-1H-imidazol-2-yl}-phenoxy)-essigsäure | 464.11 | 2.68 |
| "A51" | (4-{4-Carbamoyl-5-[3-(4-isopropyl-phenyl)-ureido]-1H-imidazol-2-yl}-phenoxy)-essigsäure | 438.17 | 2.7 |
| "A54" | (4-{4-Carbamoyl-5-[3-(4-ethyl-phenyl)-ureido]-1H-imidazol-2-yl}-phenoxy)-essigsäure | 424.15 | 2.65 |
| "A55" | (4-{4-Carbamoyl-5-[3-(2-fluor-5-trifluormethyl-phenyl)-ureido]-1H-imidazol-2-yl}-phenoxy)-essigsäure | 482.1 | 2.6 |
| "A56" | (4-{4-Carbamoyl-5-[3-(4-trifluormethyl-phenyl)-ureido]-1H-imidazol-2-ylmethoxy}-phenyl)-essigsäure | 478.13 | 2.6 |
| "A57" | (4-{4-Carbamoyl-5-[3-(4-isopropyl-phenyl)-ureido]-1H-imidazol-2-ylmethoxy}-phenyl)-essigsäure | 452.19 | 2.73 |
| "A60" | (4-{4-Carbamoyl-5-[3-(2,4-dimethyl-phenyl)-ureido]-1H-imidazol-2-ylmethoxy}-phenyl)-essigsäure | 438.17 | 2.57 |
| "A61" | (4-{4-Carbamoyl-5-[3-(4-ethyl-phenyl)-ureido]-1H-imidazol-2-ylmethoxy}phenyl)-essigsäure | 438.17 | 2.65 |
| "A62" | (4-{4-Carbamoyl-5-[3-(4-chlor-2-methyl-phenyl)-ureido]-1H-imidazol-2-ylmethoxy}-phenyl)-essigsäure | 458.12 | 2.66 |
| "A66" | 5-{4-Carbamoyl-5-[3-(4-trifluormethyl-phenyl)-ureido]-1H-imidazol-2-ylmethyl}-thiophen-2-carbonsäure | 454.07 | 2.61 |
| "A68" | 5-{4-Carbamoyl-5-[3-(4-isopropyl-phenyl)-ureido]-1H-imidazol-2-ylmethyl}-thiophen-2-carbonsäure | 428.13 | 2.63 |
| "A70" | 5-{4-Carbamoyl-5-[3-(4-chlor-phenyl)-ureido]-1H-imidazol-2-ylmethyl}-thiophen-2-carbonsäure | 420.05 | 2.53 |
| "A71" | 5-{4-Carbamoyl-5-[3-(2,4-dimethyl-phenyl)-ureido]-1H-imidazol-2-ylmethyl}-thiophen-2-carbonsäure | 414.12 | 2.48 |
| "A72" | 5-{4-Carbamoyl-5-[3-(4-ethyl-phenyl)-ureido]-1H-imidazol-2-ylmethyl}-thiophen-2-carbonsäure | 414.12 | 2.54 |
| "A74" | 5-{4-Carbamoyl-5-[3-(4-chlor-2-methyl-phenyl)-ureido]-1H-imidazol-2-ylmethyl}-thiophen-2-carbonsäure | 434.06 | 2.49 |
| "A93" | 4-{4-Carbamoyl-5-[3-(4-trifluormethoxy-phenyl)-ureido]-1H-imidazol-2-ylmethyl}-benzoesäure | 464.11 | 2.61 |
| "A97" | aus "A96" durch Reduktion mit Fe-Pulver/NH₄Cl: 2-(4-Amino-benzyl)-5-[3-(4-trifluormethyl-phenyl)-ureido]-1H-imidazol-4-carbonsäure-amid | 419.14 | 2.39 |
| "A102" | 4-[4-Carbamoyl-5-(3-thiophen-2-yl-ureido)-1H-imidazol-2-ylmethyl]-benzoesäure | 386.08 | 2.27 |

### Beispiel 2

Die Synthese von 2-[4-(4-Pyridin-2-ylmethyl-piperazin-1-carbonyl)-benzyl]-5-[3-(4-trifluormethyl-phenyl)-ureido]-1H-imidazol-4-carbonsäure-amid ("A76") erfolgt analog nachstehendem Schema 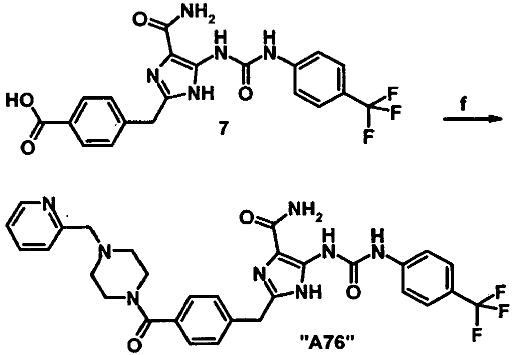
f. Verbindung 7 (100 mg, 0.2 mmol) wird in 10 ml DMF und 0.1 ml DIPEA gelöst. Nun werden EDCI (64 mg, 0.3 mmol) und HOBT (60.4 mg, 0.4 mmol) zugegeben und dann mit 1-(2-Pyridylmethyl)-piperazin (44 mg, 0.2 mmol) versetzt. Der Reaktionsansatz wird 16 h bei RT gerührt. Dann werden alle flüchtigen Bestandteile im Vakuum entfernt und aus dem Rückstand mittels präparativer HPLC das gewünschte Produkt "A76" als gelber Feststoff isoliert (57 mg, 39%).

Analog werden die nachstehenden Verbindungen erhalten

| Verbindung Nr. | Name und/oder Struktur | ESI [M+H]⁺ | HPLC (RT in min) Methode |
|---|---|---|---|
| "A84" | 5-[3-(4-Butyl-phenyl)-ureido]-2-[5-(methylphenyl-carbamoyl)-furan-2-ylmethyl]-1H-imidazol-4-carbonsäure-amid | | |
| | 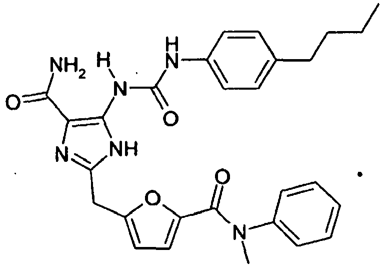 | 515.23 | 2.98 |
| "A85" | 5-[3-(4-Butyl-phenyl)-ureido]-2-[5-(4-methylpiperazin-1-carbonyl)-furan-2-ylmethyl]-1H-imidazol-4-carbonsäure-amid | 508.26 | 2.58 |
| "A90" | 5-[3-(4-Butyl-phenyl)-ureido]-2-[5-(methyl-propyl-carbamoyl)-furan-2-ylmethyl]-1H-imidazol-4-carbonsäure-amid | 481.25 | 2.91 |
| "A91" | 5-[3-(4-Butyl-phenyl)-ureido]-2-{5-[4-(2-hydroxy-ethyl)-piperidin-1-carbonyl]-furan-2-ylmethyl}-1H-imidazol-4-carbonsäure-amid | 537.27 | 2.73 |
| "A94" | 5-[3-(4-Butyl-phenyl)-ureido]-2-[4-(morpholin-4-carbonyl)-benzyl]-1H-imidazol-4-carbonsäure-amid | 505.25 | 2.75 |

Die nachfolgenden Verbindungen können mit dem Fachmann bekannten Methoden hergestellt werden. Vorzugsweise werden sie nach den Synthese-Methoden aus Beispiel 1 und 2 der vorgenannten Verbindungen hergestellt:

| Verbindung Nr. | Name und/oder Struktur | ESI [M+H]⁺ | HPLC (RT in min) Methode |
|---|---|---|---|
| "B1" | 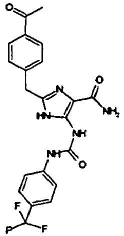 | 446 | |
| | 2-(4-Acetyl-benzyl)-5-[3-(4-trifluoromethyl-phenyl)-ureido]-1 H-imidazol-4- carbonsäure amid | | |
| "B7" | 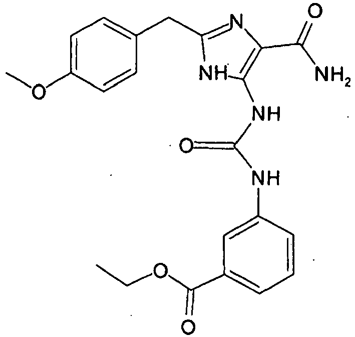 | 438 | 2.65 |
| | 3-{3-[5-Carbamoyl-2-(4-methoxy-benzyl)-3H-imidazol-4-yl]-ureido}- benzoesäure ester | | |
| "B10" | 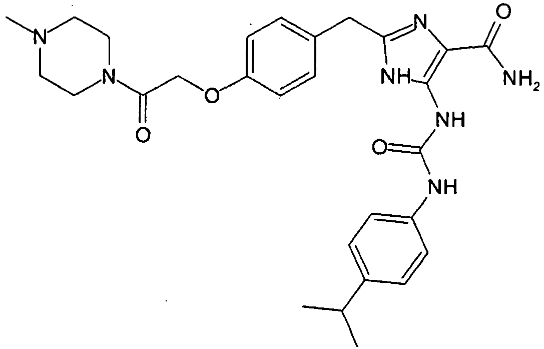 | 535 | 2.44 |
| | 5-[3-(4-Isopropyl-phenyl)-ureido]-2-{4-[2-(4-methyl-piperazin-1-yl)-2-oxo-ethoxy]-benzyl}-1H-imidazol-4 carbonsäure amid | | |
| "B11" | 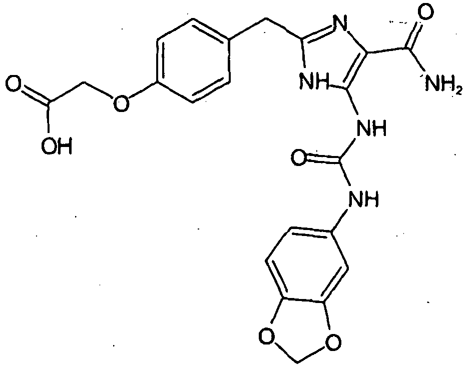 | 454 | 2.29 |
| | {4-[5-(3-Benzo[1,3]dioxol-5-yl-ureido)-4-carbamoyl-1H-imidazol-2-ylmethyl]-phenoxy}-essigsäure | | |
| "B12" | 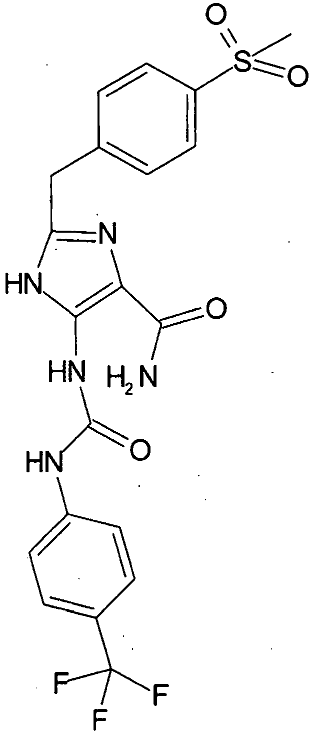 | 482 | |
| | 2-(4-Methansulfonyl-benzyl)-5-[3-(4-trifluoromethyl-phenyl)-ureido]-1H-imidazol-4-carbonsäure amid | | |
| "B19" | 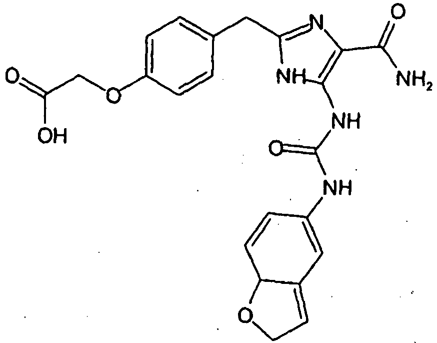 | 452 | 2.24 |
| | (4-{4-Carbamoyl-5-[3-(2,7a-dihydro-benzofuran-5-yl)-ureido]-1H-imidazol-2-ylmethyl}-phenoxy)-essigsäure | | |
| "B20" | 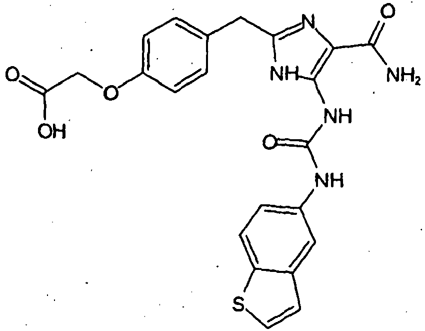 | 466 | 2.46 |
| | {4-[5-(3-Benzo[b]thiophen-5-yl-ureido)-4-carbamoyl-1H-imidazol-2-ylmethyl]-phenoxy}-essigsäure | | |
| "B25" | 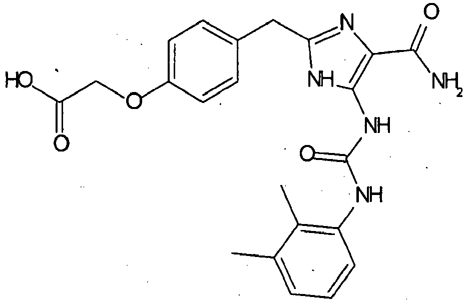 | 438 | 2.39 |
| | (4-{4-Carbamoyl-5-[3-(2,3-dimethyl-phenyl)-ureido]-1H-imidazol-2-ylmethyl}-phenoxy)-essigsäure | | |
| "B28" | 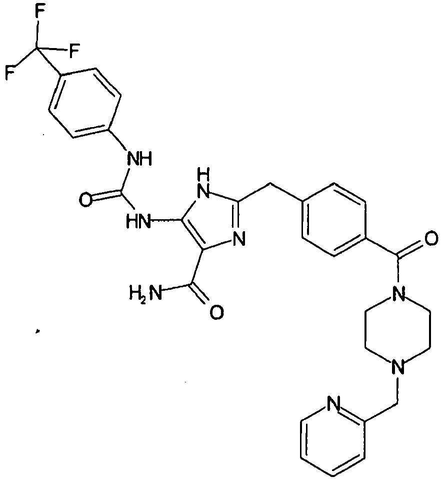 | 608 | 2.45 |
| | 2-[4-(4-Pyridin-2-ylmethyl-piperazin-1-carbonyl)-benzyl]-5-[3-(4-trifluoromethyl-phenyl)-ureido]-1H-imidazol-4- carbonsäure amid | | |
| "B30" | 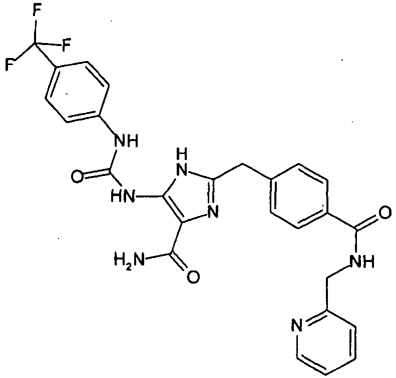 | 538 | 2.45 |
| | 2-{4-[(Pyridin-2-ylmethyl)-carbamoyl]-benzyl}-5-[3-(4-trifluoromethyl-phenyl)-ureido]-1H-imidazol-4- carbonsäure amid | | |
| "B31" | 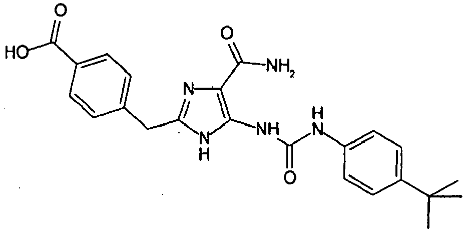 | 436 | 2.61 |
| | 4-{5-[3-(4-tert-Butyl-phenyl)-ureido]-4-carbamoyl-1 H-imidazol-2-ylmethyl}- benzoesäure | | |
| "B33" | 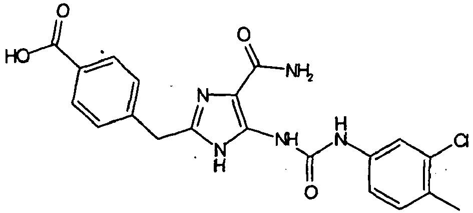 | 429 | 2.57 |
| | 4-{4-Carbamoyl-5-[3-(3-chloro-4-methyl-phenyl)-ureido]-1H-imidazol-2-ylmethyl}- benzoesäure | | |
| "B34" | 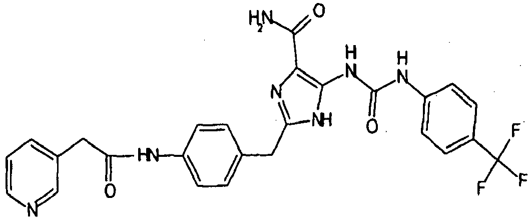 | 538 | 2.51 |
| | 2-[4-(2-Pyridin-3-yl-acetylamino)-benzyl]-5-[3-(4-trifluoromethyl-phenyl)-ureido]-1H-imidazol-4-carbonsäure amid | | |
| "B35" | 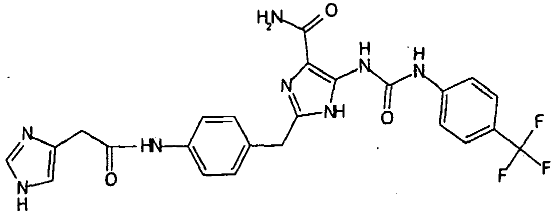 | 527 | 2.49 |
| | 2-[4-(2-1H-Imidazol-4-yl-acetylamino)-benzyl]-5-[3-(4-trifluoromethyl-phenyl)-ureido]-1H-imidazol-4- carbonsäure amid | | |
| "B36" | 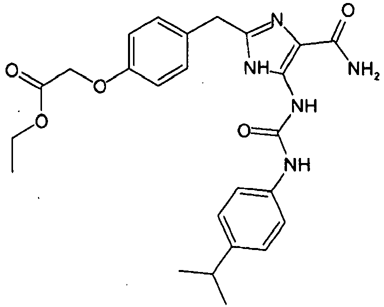 | 481 | 2,81 |
| | (4-{4-Carbamoyl-5-[3-(4-isopropyl-phenyl)-ureido]-1H-imidazol-2-ylmethyl}-phenoxy)-essigsäure ethyl ester | | |
| "B44" | 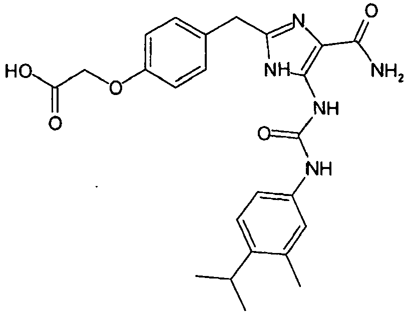 | 467 | 2.61 |
| | (4-{4-Carbamoyl-5-[3-(4-isopropyl-3-methyl-phenyl)-ureido]-1H-imidazol-2-ylmethyl}-phenoxy)- essigsäure | | |
| "B51" | 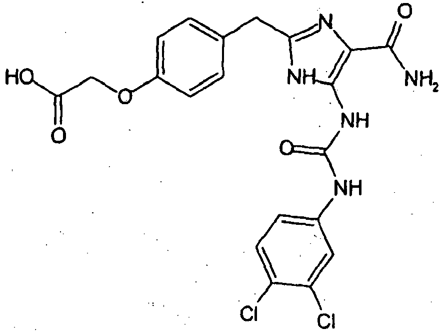 | 479 | 2.59 |
| | (4-{4-Carbamoyl-5-[3-(3,4-dichloro-phenyl)-ureido]-1H-imidazol-2-ylmethyl}-phenoxy)-essigsäure | | |
| "B52" | 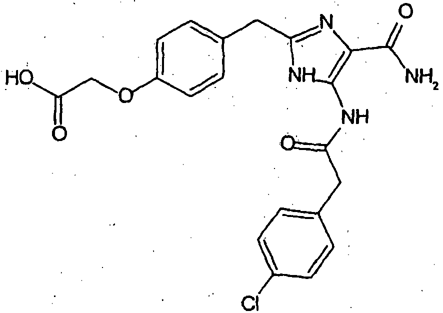 | 444 | 2.39 |
| | (4-{4-Carbamoyl-5-[2-(4-chloro-phenyl)-acetylamino]-1H-imidazol-2-ylmethyl}-phenoxy)-essigsäure | | |
| "B53" | 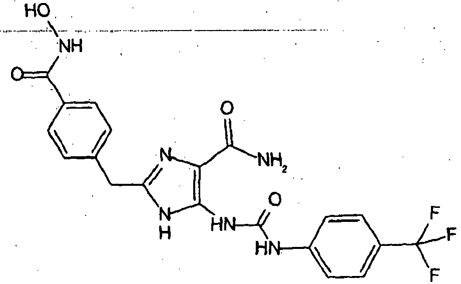 | 463 | 2.42 |
| | 2-(4-Hydroxycarbamoyl-benzyl)-5-[3-(4-trifluoromethyl-phenyl)-ureido]-1H-imidazol-4-carbonsäure amid | | |
| "B54" | 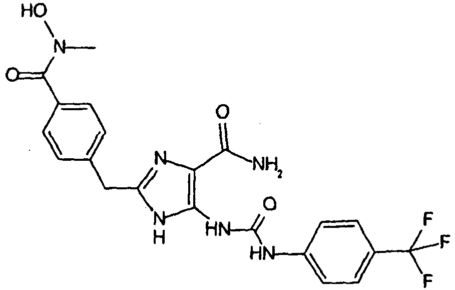 | 477 | 2.48 |
| | 2-[4-(Hydroxy-methyl-carbamoyl)-benzyl]-5-[3-(4-trifluoromethyl-phenyl)-ureido]-1H-imidazol-4-carbonsäure amid | | |
| "B55" | 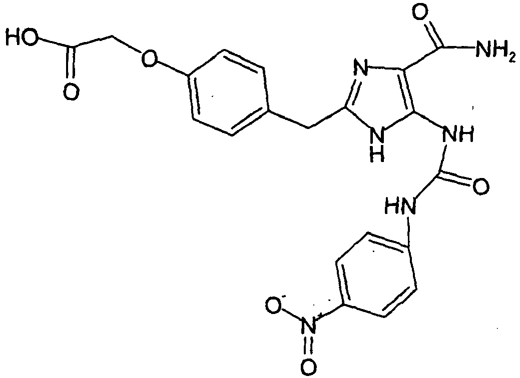 | 455 | 2.65 |
| | (4-{4-Carbamoyl-5-[3-(4-nitro-phenyl)-ureido]-1H-imidazo)-2-ylmethyl}-phenoxy)-essigsäure | | |
| "B56" | 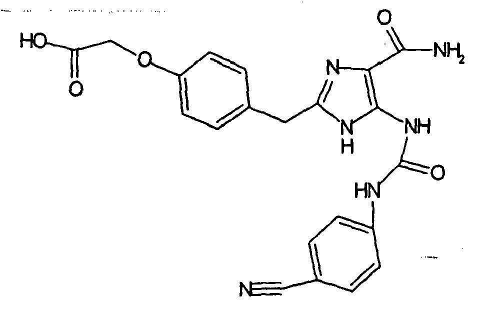 | 435 | 2.04 |
| | (4-{4-Carbamoyl-5-[3-(4-cyano-phenyl)-ureido]-1H-imidazol-2-ylmethyl}-phenoxy)- essigsäure | | |
| "B57" | 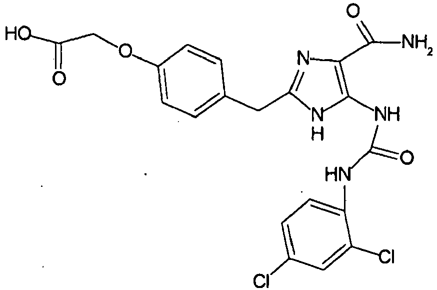 | 479 | 3.02 |
| | (4-{4-Carbamoyl-5-[3-(2,4-dichloro-phenyl)-ureido]-1H-imidazol-2-ylmethyl}-phenoxy)-essigsäure | | |
| "B62" | 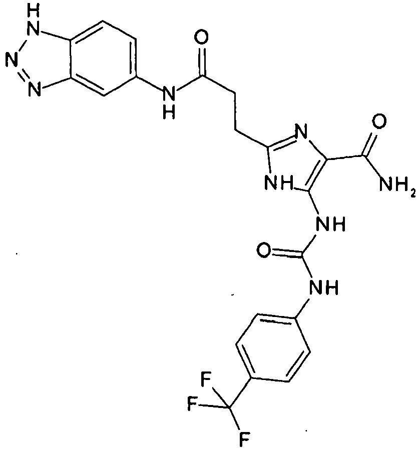 | 502 | 2.70 |
| | 2-[2-(1H-Benzotriazol-5-ylcarbamoyl)-ethyl]-5-[3-(4-trifluoromethyl-phenyl)-ureido]-1H-imidazol-4-carbonsäure amid | | |
| "B64" | 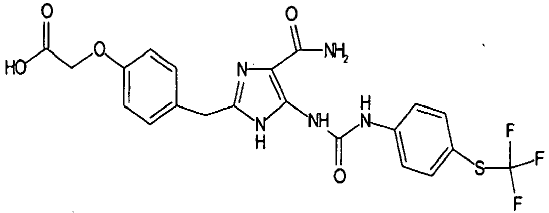 | 510 | 3.06 |
| | (4-{4-Carbamoyl-5-[3-(4-trifluoromethylsulfanyl-phenyl)-ureido]-1H-imidazol-2-ylmethyl}-phenoxy)- essigsäure | | |
| "B65" | 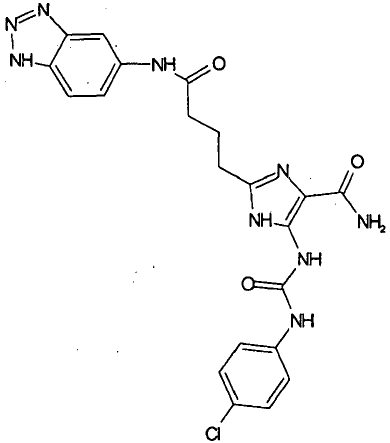 | 483 | 2.53 |
| | 2-[3-(1H-Benzotriazol-5-ylcarbamoyl)-propyl]-5-[3-(4-chloro-phenyl)-ureido]-1H-imidazol-4-carbonsäure | | |
| "B66" | 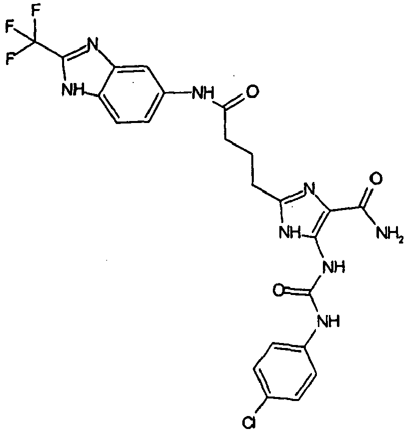 | 550 | 2.71 |
| | 5-[3-(4-Chloro-phenyl)-ureido]-2-[3-(2-trifluoromethyl-1H-benzimidazol-5-ylcarbamoyl)-propyl]-1H-imidazol-4- carbonsäure | | |
| "B67" | 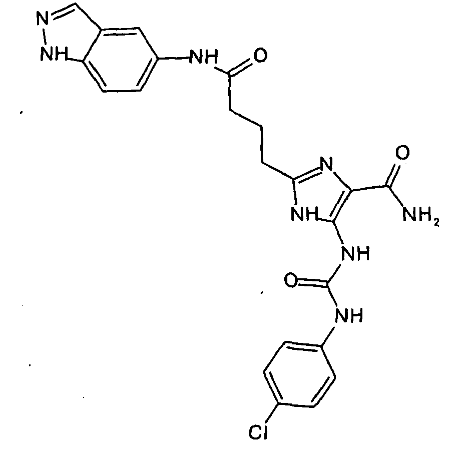 | 482 | 2.55 |
| | 5-[3-(4-Chloro-phenyl)-ureido]-2-[3-(1H-indazol-5-ylcarbamoyl)-propyl]-1H-imidazol-4-carbonsäure | | |
| **"**B70**"** | 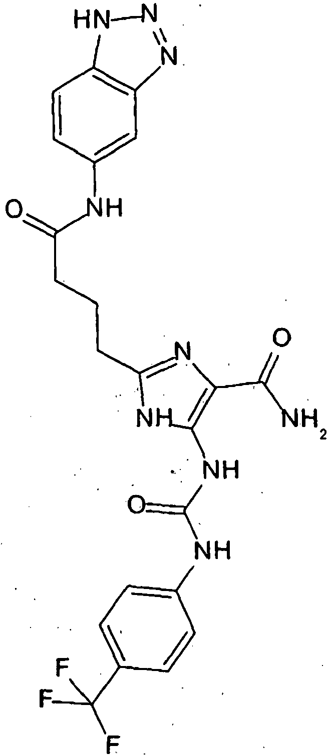 | 516 | 2.70 |
| | 2-[3-(1H-Benzotriazol-5-ylcarbamoyl)-propyl]-5-[3-(4-trifluoromethyl-phenyl)-ureido]-1H-imidazol-4-carbonsäure | | |
| "B71" | 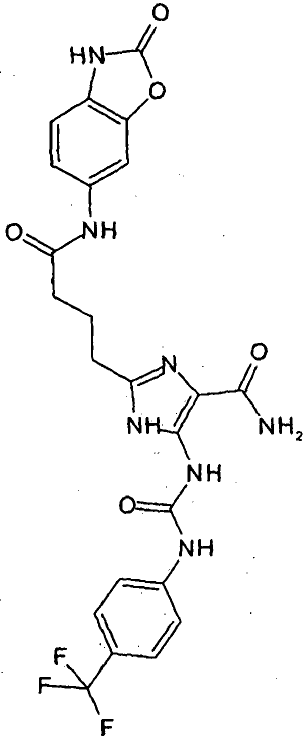 | 532 | 2.72 |
| | 2-[3-(2-Oxo-2,3-dihydro-benzoxazol-6-ylcarbamoyl)-propyl]-5-[3-(4-trifluoromethyl-phenyl)-ureido]-1H-imidazol-4-carbonsäure | | |
| "B72" | 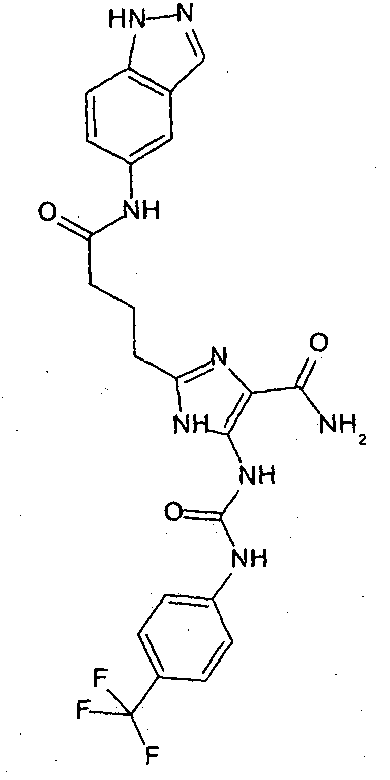 | 515 | 2.72 |
| | 2-[3-(1H-Indazol-5-ylcarbamoyl)-propyl]-5-[3-(4-trifluoromethyl-phenyl)-ureido]-1H-imidazol-4-carbonsäure | | |
| "B73" | 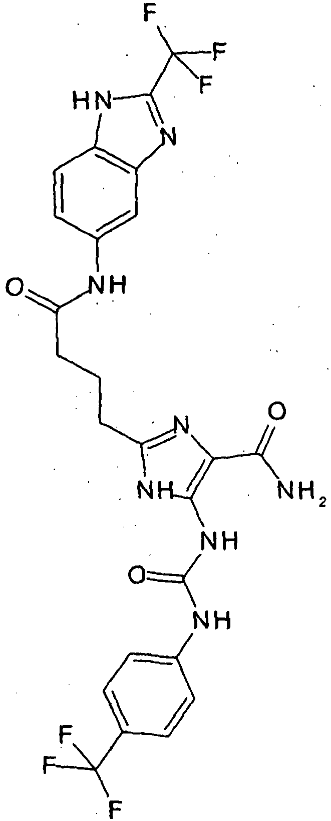 | 583 | 2.87 |
| | 2-[3-(2-Trifluoromethyl-1H-benzimidazol-5-ylcarbamoyl)-propyl]-5-[3-(4-trifluoromethyl-phenyl)-ureido]-1H-imidazol-4- carbonsäure | | |
| "B74" | 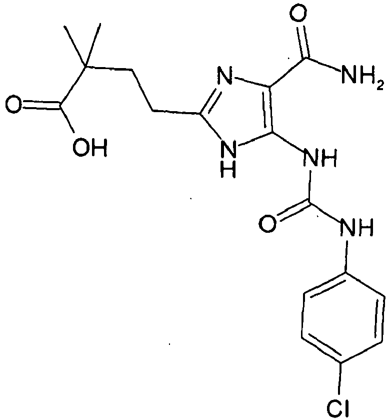 | 518 | 2.74 |
| | 2-[2-(2-Oxo-2,3-dihydro-benzoxazol-6-ylcarbamöyl)-ethyl]-5-[3-(4-trifluoromethyl-phenyl)-ureido]-1H-imidazol-4-carbonsäure | | |
| "B76" | 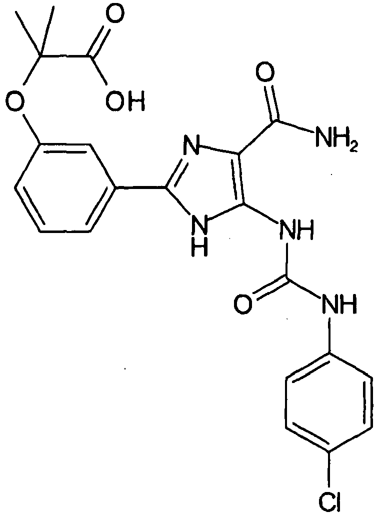 | 459 | 3.17 |
| | 2-(3-{4-Carbamoyl-5-[3-(4-chloro-phenyl)-ureido]-1H-imidazol-2-yl}-phenoxy)-2-methyl-propionsäure | | |
| "B77" | 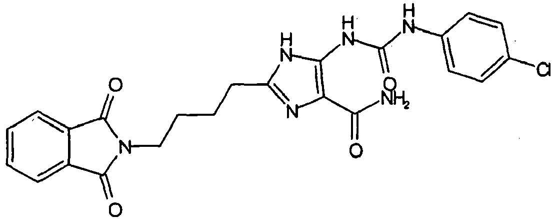 | 482 | 3.04 |
| | 5-[3-(4-Chloro-phenyl)-ureido]-2-(1,3-dioxo-1,3-dihydro-isoindol-2-ylmethyl)-1H-imidazol-4-carbonsäure-amid | | |
| "B78" | 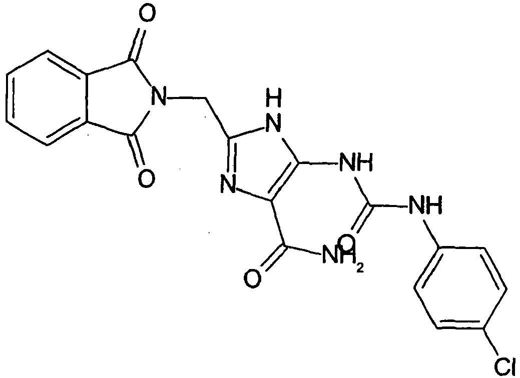 | 440 | 3.08 |
| | 5-[3-(4-Chloro-phenyl)-ureido]-2-(1,3-dioxo-1,3-dihydro-isoindol-2-ylmethyl)-1H-imidazol-4-carbonsäure-amid | | |
| "B79" | 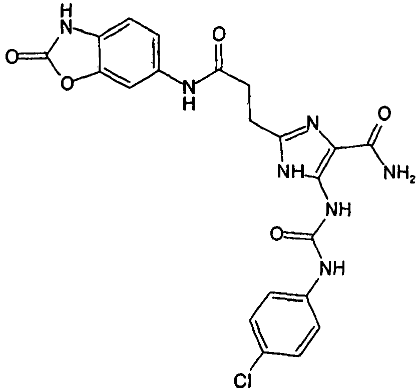 | 485 | 2.60 |
| | 5-[3-(4-Chloro-phenyl)-ureido]-2-[2-(2-oxo-2,3-dihydro-benzoxazol-6-ylcarbamoyl)-ethyl]-1H-imidazol-4-carbonsäure-amid | | |
| "B80" | 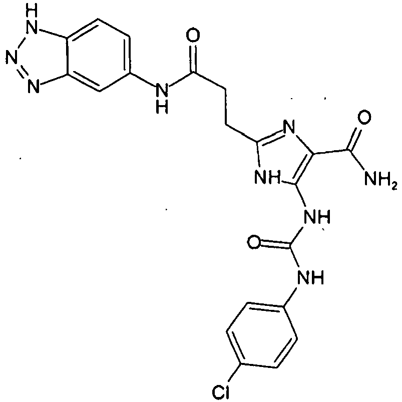 | 469 | 2.58 |
| | 2-[2-(1H-Benzotriazol-5-ylcarbamoyl)-ethyl]-5-[3-(4-chloro-phenyl)-ureido]-1H-imidazol-4-carbonsäure-amid | | |

### Pharmakologische Daten

Autotaxin-Inhibierung (Enzym Test)

**Tabelle 1**

| Verbindung Nr. | IC50 |
|---|---|
| "A2" | A |
| "A6" | A |
| "A7" | A |
| "A8" | A |
| "A9" | A |
| "A10" | A |
| "A11" | A |
| "A12" | A |
| "A15" | A |
| "A27" | A |
| "A30" | A |
| "A31" | A |
| "A38" | A |
| "A1" | A |
| "A39" | A |
| "A40" | A |
| "A41" | A |
| "A42" | A |
| "A43" | A |
| "A49" | A |
| "A50" | A |
| "A51" | A |
| "A54" | A |
| "A55" | A |
| "A56" | A |
| "A57" | A |
| "A60" | A |
| "A61" | A |
| "A62" | A |
| "A66" | A |
| "A68" | A |
| "A71" | A |
| "A72" | A |
| "A74" | A |
| "A84" | A |
| "A85" | A |
| "A91" | A |
| "A93" | A |
| "A94" | A |
| "A97" | A |
| "A102" | A |
| "A3" | B |
| "A4" | B |
| "A5" | B |
| "A13" | B |
| "A14" | B |
| "A16" | B |
| "A17" | B |
| "A18" | B |
| "A19" | B |
| "A20" | B |
| "A21" | B |
| "A22" | B |
| "A23" | B |
| "A24" | B |
| "A25" | B |
| "A26" | B |
| "A28" | B |
| "A28" | B |
| "A29" | B |
| "A32" | B |
| "A33" | B |
| "A36" | B |
| "A37" | B |
| "A44" | B |
| "A45" | B |
| "A46" | B |
| "A47" | B |
| "A48" | B |
| "A52" | B |
| "A53" | B |
| "A58" | B |
| "A59" | B |
| "A63" | B |
| "A64" | B |
| "A65" | B |
| "A67" | B |
| "A69" | B |
| "A73" | B |
| "A75" | B |
| "A77" | B |
| "A78" | B |
| "A79" | B |
| "A80" | B |
| "A81" | B |
| "A82" | B |
| "A83" | B |
| "A86" | B |
| "A87" | B |
| "A88" | B |
| "A89" | B |
| "A92" | B |
| "A95" | B |
| "A96" | B |
| "A98" | B |
| "A99" | B |
| "A100" | B |
| "A101" | B |
| "A103" | B |
| "B77" | B |
| "B78" | B |
| "B79" | B |
| "B80" | B |

| | |
|---|---|
| IC50: 1 µM-10µM=A >10 µM= B | |

### Beispiel A: Autotaxin Test (Enzym Test)

### Testbeschreibung

Die Autotaxin Aktivität wird indirekt mit dem Amplex Red Reagenz gemessen. Hierbei wird Amplex Red als fluorgenischem Indikatior für das entstandene H₂O₂ gemessen. Im Detail setzt Autotaxin das Substrat Lysophosphatidylcholin (LPC) zu Phosphocholin und Lysophosphatidylsäure (LPS) um. Nach dieser Umsetzung wird das Phosphocholin mit alkalischer Phosphatase zu inorganischem Phosphat und Cholin ungesetzt. Im nächsten Schritt wird Cholin durch Choline-Öxidase zu Betain oxidiert, wobei H₂O₂ entsteht. H₂O₂ reagiert in Gegenwart von Peroxidase (Horseradish peroxidase) mit dem Amplex Red Reagenz in eine 1:1 Stöchiometrie und bildet das hochfluoreszente Resorufin. Die Fluoreszenz wird in einem reaktionsabhängigen kinetischen Modus gemessen, damit dass fluoreszente Signale möglicher anderer fluoreszenter Stoffe, die nicht an der Reaktion beteiligt sind, herauskorrigiert werden kann.

### Testausführung

1,5 µl einer Standardlösung oder der Testsubstanzen (Substanzen mit dem Namen A(n)) in individuellen Konzentrationen gelöst in 20mM Hepes pH 7.2 mit maximal 7.7% DMSO werden zusammen mit 10 µl (16 ng) hochgereinigten recombinanten Autotaxin in einer schwarzen mit 384 Vertiefungen versehenen Mikrotiterplatte für 30 min bei 22°C vorinkubiert. Danach wird die Reaktion durch Zugabe von 5µl L-α-Lysophosphatidylcholin (LPC) gestartet, wobei die Endkonzentration von LPC 75 µM beträgt. Die Mischung wird 90 min. bei 37°C inkubiert. Nach der Inkubation wird Amplex Red Reagenz, Peroxidase (Horseradish peroxidase) und Cholin-Oxidase hinzugefügt und sofort die Fluoreszenz bei 612 nm bei einer Anregung von 485 nm in einem "Tecan Ultra multimode" Lesegerät gemessen. Die Aktivität von Autotaxin wird indirekt über den Nachweis des anfallenden H₂O₂ errechnet.

### Material:

| | |
|---|---|
| Microtiterplatte: | PS-Microplate, 384 Vertiefungen, kleines Volumen, schwarz Corning, Cat#3677 |
| Protein: | Recombinantes Autotaxin (Baculovirale Hi5 Expression) |
| Substrat: | L-α-Lysophosphatidylcholin (Hühnerei)); Avanti Polar Lipids # 830071P |
| Standard: | C14 LPA, Avanti Potar Lipids, Cat# 857120P |
| Nachweis Reagenz: | Amplex Red Reagenz; Inivitrogen # A12222; gelöst in 1.923 ml of DMSO Peroxidase Type VI-A (horseradish) von Sigma # P6782; gelöst in 7,45 ml Test Puffer, Choline-Oxidase ; Sigma # C5896; gelöst in 2,47 ml Test Puffer |
| Nachweis Reagenz Mix: | 1:100 Verdünnung von Amplex Red Regenzt in Test Puffer |
| Test Puffer: | 200 mM Tris-HCl, Merck, Cat # 1.08219, pH 7.9, 0.1 % BSA, lipidfrei, Roche Cat#775835 |

Die nachfolgenden Beispiele betreffen Arzneimittel:

### Beispiel B: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektions-glas enthält 5 mg Wirkstoff.

### Beispiel C: Suppositorien

Man schmilzt ein Gemisch von 20 g eines Wirkstoffes der Formel I mit 100 g Sojalecithin und 1400 g Kakaobutter, gießt in Formen und lässt erkalten. Jedes Suppositorium enthält 20 mg Wirkstoff.

### Beispiel D: Lösung

Man bereitet eine Lösung aus 1 g eines Wirkstoffes der Formel I, 9,38 g NaH₂PO₄ - 2 H₂O, 28,48 g Na₂HPO4 · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel E: Salbe

Man mischt 500 mg eines Wirkstoffes der Formel I mit 99,5 g Vaseline unter aseptischen Bedingungen.

### Beispiel F: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpresst, derart, dass jede Tablette 10 mg Wirkstoff enthält.

### Beispiel G: Dragees

Analog Beispiel E werden Tabletten gepresst, die anschließend in üblicher Weise mit einem Überzug aus Saccharose, Kartoffelstärke, Talk, Tragant und Farbstoff überzogen werden.

### Beispiel H: Kapseln

2 kg Wirkstoff der Formel I werden in üblicher Weise in Hartgelatinekapseln gefüllt, so dass jede Kapsel 20 mg des Wirkstoffs enthält.

### Bespiel I: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
| Nr. | Name und/oder Struktur |
|---|---|
| "A1" | (4-{4-Carbamoyl-5-[3-(4-isopropyl-phenyl)-ureido]-1H-imidazol-2-ylmethyl}-phenoxy)-essigsäure |
| "A2" | 4-{4-Carbamoyl-5-[3-(4-isopropyl-phenyl)-ureido]-1H-imidazol-2-ylmethyl}-benzoesäure |
| | 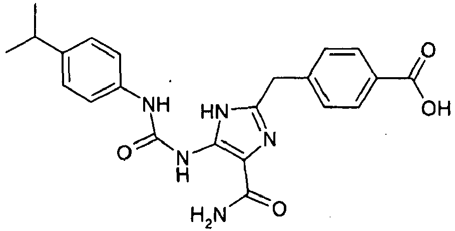 |
| "A6" | 4-{4-Carbamoyl-5-[3-(4-chlor-phenyl)-ureido]-1H-imidazol-2-ylmethyl}-benzoesäure |
| "A7" | 4-{4-Carbamoyl-5-[3-(4-ethyl-phenyl)-ureido]-1H-imidazol-2-ylmethyl}-benzoesäure |
| "A8" | 4-{4-Carbameyl-5-[3-(3-chler-2-methyl-phenyl)-ureido]-1H-imidazol-2-ylmethyl}-benzoesäure |
| "A9" | 4-{5-[3-(4-Butoxy-phenyl)-ureido]-4-carbamoyl-1H-imidazol-2-ylmethyl}-benzoesäure |
| "A10" | 5-{4-Carbamoyl-5-[3-(4-trifluormethyl-phenyl)-ureido]-1H-imidazol-2-ylmethyl}-furan-2-carbonsäure |
| | 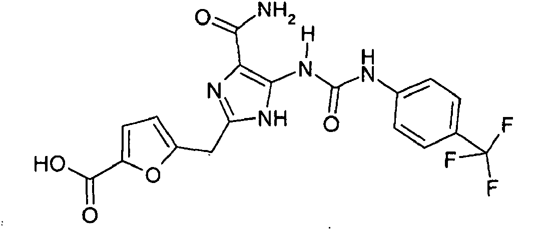 |
| "A11" | 5-{4-Carbamoyl-5-[3-(4-ethyl-phenyl)-ureido]-1H-imidazol-2-ylmethyl}-furan-2-carbonsäure |
| "A12" | 5-{4-Carbamoyl-5-[3-(4-isopropyl-phenyl)-ureido]-1H-imidazol-2-ylmethyl}-furan-2-carbonsäure |
| "A15" | 4-{4-Carbamoyl-5-[3-(4-trifluormethyl-phenyl)-ureido]-1H-imidazol-2-ylmethyl}-benzoesäure |
| "A27" | 5-[4-Carbamoyl-5-(3-phenethyl-ureido)-1H-imidazol-2-ylmethyl]-furan-2-carbonsäure |
| "A30" | 5-{4-Carbamoyl-5-[3-(4-chlor-phenyl)-ureido)-1H-imidazol-2-ylmethyl}-furan-2-carbonsäure |
| "A31" | 5-{4-Carbameyl-5-[3-(2 ,4,6-trimethyl-phenyl)-ureida]-1H-imidazol-2-ylmethyl}-furan-2-carbonsäure |
| "A34" | 5-{4-Carbamoyl-5-[3-(2-fluor-5-trifluormethyl-phenyl)-ureido]-1H-imidazol-2-ylmethyl}-furan-2-carbonsäure |
| "A35" | 5-{4-Carbamoyl-5-[3-(4-chlor-2-trifluormethyl-phenyl)-ureido]-1H-imidazol-2-ylmethyl}-furan-2-carbonsäure |
| "A38" | (4-{4-Carbamoyl-5-[3-(4-trifluormethyl-phenyl)-ureido]-1H-imidazol-2-ylmethyl}-phenoxy)-essigsäure |
| "A39" | (4-{5-[3-(4-Butyl-phenyl)-ureido]-4-carbamoyl-1H-imidazol-2-ylmethyl}-phenoxy)-essigsäure |
| "A40" | {4-[4-Carbamoyl-5-(3-p-tolyl-ureido)-1H-imidazol-2-yfmethyl]-phenoxy}-essigsäure |
| "A41" | (4-{4-Carbamoyl-5-[3-(4-chlor-phenyl)-ureido]-1H-imidazol-2-ylmethyl}-phenoxy)-essigsäure |
| "A42" | (4-{4-Carbamoyl-5-[3-(4-ethyl-phenyl)-ureido]-1H-imidazol-2-ylmethyl}-phenoxy)-essigsäure |
| "A43" | (4-{4-Carbamoyl-5-[3-(4-penty)-phenyl)-ureido]-1H-imidazo)-2-ylmethyl}-phenoxy)-essigsäure |
| "A49" | (4-{4-Carbamoyl-5-[3-(3-trifluormethyl-phenyl)-ureido]-1H-imidazol-2-yl}-phenoxy)-essigsäure |
| "A50" | (4-{4-Carbamoyl-5-[3-(4-trifluormethyl-phenyl)-ureido]-1H-imidazol-2-yl}-phenoxy)-essigsäure |
| "A51" | (4-{4-Carbamoyl-5-[3-(4-isopropyl-phenyl)-ureido]-1H-imidazol-2-yl}-phenoxy)-essigsäure |
| "A54" | (4-{4-Carbamoyl-5-[3-(4-ethyl-phenyl)-ureido]-1H-imidazol-2-yl}-phenoxy)-essigsäure |
| "A55" | (4-{4-Carbamoyl-5-[3-(2-fluor-5-trifluormethyl-phenyl)-ureido]-1H-imidazol-2-yl}-phenoxy)-essigsäure |
| "A56" | (4-{4-Carbamoyl-5-[3-(4-trifluormethyl-phenyl)-ureido]-1H-imidazol-2-ylmethoxy}-phenyl)-essigsäure |
| "A57" | (4-{4-Carbamoyl-5-[3-(4-isopropyl-phenyl)-ureido]-1H-imidazol-2-ylmethoxy}-phenyl)-essigsäure |
| "A60" | (4-{4-Carbamoyl-5-[3-(2,4-dimethyl-phenyl)-ureido]-1H-imidazol-2-ylmethoxy}-phenyl)-essigsäure |
| "A61" | (4-{4-Carbamoyl-5-[3-(4-ethyl-phenyl)-ureido]-1H-imidazol-2-ylmethoxy}-phenyl)-essigsäure |
| "A62" | (4-{4-Carbamoyl-5-[3-(4-chlor-2-methyl-phenyl)-ureido]-1H-imidazol-2-ylmethoxy}-phenyl)-essigsäure |
| "A66" | 5-{4-Carbamoyl-5-[3-(4-trifluormethyl-phenyl)-ureido]-1H-imidazol-2-ylmethyl}-thiophen-2-carbonsäure |
| "A68" | 5-{4-Carbamoyl-5-[3-(4-isopropyl-phenyl)-ureido]-1H-imidazol-2-ylmethyl}-thiophen-2-carbonsäure |
| "A70" | 5-{4-Carbamoyl-5-[3-(4-chlor-phenyl)-ureido]-1H-imidazol-2-ylmethyl}-thiophen-2-carbonsäure |
| "A71" | 5-(4-Carbamoyl-5-[3-(2,4-dimethyl-phenyl)-ureido]-1H-imidazol-2-ylmethyl}-thiophen-2-carbonsäure |
| "A72" | 5-{4-Carbamoyl-5-[3-(-ethyl-phenyl)-ureido]-1H-imidazal-2-ylmethyl}-thiophen-2-carbonsäure |
| "A74" | 5-{4-Carbamoyl-5-[3-(4-chlor-2-methyl-phenyl)-ureido]-1H-imidazol-2-ylmethyl}-thiophen-2-carbonsäure |
| "A84" | 5-[3-(4-Butyl-phenyl)-ureido]-2-[5-(methyl-phenylcarbamoyl)-furan-2-ylmethyl]-1H-imidazol-4-carbonsäure-amid |
| | 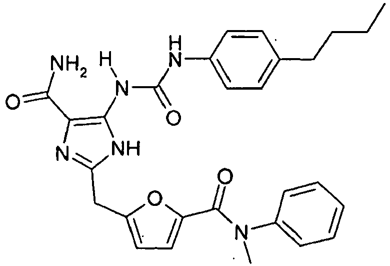 |
| "A85" | 5-[3-(4-Butyl-phenyl)-ureido]-2-[5-(4-methyl-piperazin-1-carbonyl)-furan-2-ylmethyl]-1H-imidazol-4-carbonsäure-amid |
| "A91" | 5-[3-(4-Butyl-phenyl)-ureido]-2-{5-[4-(2-hydroxy-ethyl)-piperidin-1-carbonyl]-furan-2-ylmethyl}-1H-imidazol-4-carbonsäure-amid |
| "A93" | 4-{4-Carbamoyl-5-[3-(4-trifluormethoxy-phenyl)-ureido]-1H-imidazol-2-ylmethyl}-benzoesäure |
| "A94" | 5-[3-(4-Butyl-phenyl)-ureido]-2-[4-(morpholin-4-carbonyl)-benzyl]-1H-imidazol-4-carbonsäure-amid |
| "A97" | aus "A96" durch Reduktion mit Fe-Puler/NH₄Cl: 2-(4-Amino-berizyl)-5-[3-(4-trifluormethyl-phenyl)-ureido]-1H-imidazol-4-carbonsäure-amid |
| "A102" | 4-[4-Carbamoyl-5-(3-thiophen-2-yl-ureido)-1H-imidazol-2-ylmethyl]-benzoesäure |
| "B2" | 2-[4-(3,5-Dimethyl-pyrazol-1-carbonyl)-benzyl]-5-[3-(4-trifluoromethyl-phenyl)-ureido]-1H-imidazol-4- carbonsäure amid, |
| "B4" | 5-[3-(4-Isopropyl-phenyl)-ureido]-2-(4-methoxy-benzyl)-1H-imidazol-4- carbonsäure amid, |
| "B7" | 3-{3-[5-Carbamoyl-2-(4-methoxy-benzyl)-3H-imidazol-4-yl]-ureido}- benzoesäure ester, |
| "B10" | 5-[3-(4-Isopropyl-phenyl)-ureido]-2-{4-[2-(4-methyl-piperazin-1-yl)-2-oxo-ethoxyl-benzyl}-1H-imidazol-4 carbonsäure amid, |
| "B11" | {4-[5-(3-Benzo[1,3]dioxol-5-yl-ureido)-4-carbamoyl-1H-imidazol-2-ylmethyl]-phenoxy}-essigsäure, |
| "B12" | 2-(4-Methansulfonyl-benzyl)-5-[3-(4-trifluoromethyl-phenyl)-ureido]-1H-imidazol-4- carbonsäure amid, |
| "B19" | (4-{4-Carbamoyl-5-[3-(2,7a-dihydro-benzofuran-5-yl)-ureido]-1H-imidazol-2-ylmethyl}-phenoxy)- essigsäure, |
| "B20" | ( {4-[5-(3-Benzo[b]thiophen-5-yl-ureido)-4-carbamoyl-1H-imidazol-2-ylmethyl]-phenoxy}- essigsäure, |
| "B25" | (4-{4-Carbamoyl-5-[3-(2,3-dimethyl-phenyl)-ureido]-1H-imidazol-2-ylmethyl}-phenoxy)- essigsäure, |
| "B28" | 2-[4-(4-Pyridin-2-ylmethyl-piperazin-1-carbonyl)-benzyl]-5-[3-(4-trifluoromethyl-phenyl)-ureido]-1H-imidazol-4-carbonsäure, amid |
| "B30" | 2-{4-[(Pyridin-2-ylmethyl)-carbamoyl]-benzyl}-5-[3-(4-trifluoromethyl-phenyl)-ureido]-1H-imidazol-4- carbonsäure amid, |
| "B31" | 4-{5-[3-(4-tert-Butyl-phenyl)-ureido]-4-carbamoyl-1H-imidazol-2-ylmethyl}-benzoesäure, |
| "B33" | |
| | 4-{4-Carbamoyl-5-[3-(3-chloro-4-methyl-phenyl)-ureido]-1H-imidazol-2-ylmethyl}- benzoesäure, |
| "B34" | 2-[4-(2-Pyridin-3-yl-acetylamino)-benzyl]-5-[3-(4-trifluoromethyl-phenyl)-ureido]-1H-imidazol-4-carbonsäure amid, |
| "B35" | 2-[4-(2-1H-Imidazol-4-yl-acetylamino)-benzyl]-5-[3-(4-trifluoromethyl-phenyl)-ureido]-1H-imidazol-4-carbonsäure amid, |
| "B36" | (4-{4-Carbamoyl-5-[3-(4-isopropyl-phenyl)-ureido]-1H-imidazol-2-ylmethyl}-phenoxy)-essigsäure ethyl ester; |
| "B44" | (4-{4-Carbamoyl-5-[3-(4-isopropyl-3-methyl-phenyl)-ureido]-1H-imidazol-2-ylmethyl}-phenoxy)-essigsäure, |
| "B51" | (4-{4-Carbamoyl-5-[3-(3,4-dichloro-phenyl)-ureido]-1H-imidazol-2-ylmethyl}-phenoxy)-essigsäure, |
| "B52" | (4-{4-Carbamoyl-5-[2-(4-chloro-phenyl)-acetylamino]-1H-imidazol-2-ylmethyl}-phenoxy)-essigsäure, |
| "B53" | 2-(4-Hydroxycarbamoyl-benzyl)-5-[3-(4-trifluoromethyl-phenyl)-ureide]-1H-imidazol-4- carbonsäure amid, |
| "B54" | 2-[4-(Hydroxy-methyl-carbamoyl)-benzyl]-5-[3-(4-trifluoromethyl-phenyl)-ureido]-1H-imidazol-4- carbonsäure amid, |
| "B55" | (4-{4-Carbamoyl-5-[3-(4-nitro-phenyl)-ureide]-1H-imidazal-2-ylmethyl}-phenoxy)-essigsäure |
| "B56" | (4-{4-Carbamoyl-5-[3-(4-cyano-phenyl)-ureidol-1H-imidazol-2-ylmethyl}-phenoxy)- essigsäure, |
| "B57" | (4-{4-Carbamoyl-5-[3-(2,4-dichloro-phenyl)-ureido]-1H-imidazol-2-ylmethyl}-phenoxy)-essigsäure, |
| "B62" | 2-[2-(1H-Benzotriazol-5-ylcarbamoyl)-ethyl]-5-[3-(4-trifluoromethyl-phenyl)-ureido]-1H-imidazol-4-carbonsäure amid, |
| "B64" | (4-{4-Carbamoyl-5-[3-(4-trifluoromethylsulfanyl-phenyl)-ureido]-1H-imidazol-2-ylmethyl}-phenoxy)-essigsäure, |
| "B65" | 2-[3-(1H-Benzotriazol-5-ylcarbamoyl)-propyl]-5-[3-(4-chlorophenyl)-ureido]-1H-imidazol-4-carbonsäure, |
| "B66" | 5-[3-(4-Chloro-phenyl)-ureido]-2-[3-(2-trifluoromethyl-1H-benzimidazol-5-ylcarbamoyl)-propyl]-1H-imidazol-4-carbonsäure, |
| "B67" | 5-[3-(4-Chloro-phenyl)-ureido]-2-[3-(1H-indazol-5-ylcarbamoyl)-propyl]-1H-imidazol-4-carbonsäure, |
| "B70" | 2-[3-(1H-Benzotriazol-5-ylcarbamoyl)-propyl]-5-[3-(4-trifluoromethyl-phenyl)-ureido]-1H-imidazol-4-carbonsäure, |
| "B71" | 2-[3-(2-Oxo-2,3-dihydro-benzoxazol-6-ylcarbamoyl)-propyl]-5-[3-(4-trifluoromethyl-phenyl)-ureido]-1H-imidazol-4-carbonsäure, |
| "B72" | 2-[3-(1H-Indazol-5-ylcarbamoyl)-propyl]-5-[3-(4-trifluoromethyl-phenyl)-ureido]-1H-imidazol-4-carbonsäure, |
| "B73" | 2-[3-(2-Trifluoromethyl-1H-benzimidazol-5-ylcarbamoyl)-propyl]-5-[3-(4-trifluoromethyl-phenyl)-ureido]-1H-imidazol-4-carbonsäure, |
| "B74" | 2-[2-(2-Oxo-2, 3-dihydro-benzoxazol-6-ylcarbamoyl)-ethyl]-5-[3-(4-trifluoromethyl-phenyl)-ureido]-1H-imidazol-4-carbonsäure, |
| "B76" | 2-(3-{4-Carbamoyl-5-[3-(4-chloro-phenyl)-ureido]-1H-imidazol-2-yl}-phenoxy)-2-methyl-propionsäure, |
| "B77" | 5-[3-(4-Chloro-phenyl)-ureido]-2-(1,3-dioxo-1,3-dihydro-isoindol-2-ylmethyl)-1H-imidazol-4-carbonsäure-amid, |
| "B78" | 5-[3-(4-Chloro-phenyl)-ureido]-2-(1,3-dioxo-1,3-dihydro-isoindol-2-ylmethyl)-1H-imidazol-4- carbonsäure-amid, |
| "B79" | 5-[3-(4-Chloro-phenyl)-ureido]-2-[2-(2-oxo-2, 3-dihydro-benzoxazol-6-ylcarbamoyl)-ethyl]-1H-imidazol-4-carbonsäure-amid oder |
| "B80" | 2-[2-(1H-Benzotriazol-5-ylcarbamoyl)-ethyl]-5-[3-(4-chlorophenyl)-ureido]-1H-imidazol-4-carbonsäure-amid, |
sowie ihre pharmazeutisch verwendbaren, Tautomere, Salze und Stereoisomere, einschließlich deren Mischungen in allen Verhältnissen.

2. Arzneimittel, enthaltend mindestens eine Verbindung nach Anspruch 1 und/oder ihre pharmazeutisch verwendbaren Salze, Solvate, Tautomere und Stereoisomeren, einschließlich deren Mischungen in allen Verhältnissen, sowie gegebenenfalls Träger- und/oder Hilfsstoffe.

3. Verwendung von Verbindungen nach Anspruch 1 sowie ihrer pharmazeutisch verwendbaren, Salze, Solvate, Tautomeren und Stereoisomeren, einschließlich deren Mischungen in allen Verhältnissen, zur Herstellung eines Arzneimittels zur Behandlung von Krankheiten, bei denen die Hemmung, Regulierung und/oder Modulation der Phosphodiesterase bzw. Lysophospholipase Autotaxin eine Rolle spielt.

4. Verwendung von Verbindungen nach Anspruch 1, zur Herstellung eines Arzneimittels zur Behandlung und Prophylaxe von Krebskrankheiten.

5. Verwendung nach Anspruch 4, wobei die Krebskrankheiten mit einem Tumor aus der Gruppe der Tumoren des Plättenepithel, der Blasen, des Magens, der Nieren, von Kopf und Hals, des Ösophagus, des Gebärmutterhals, der Schilddrüse; des Darm, der Leber, des Gehirns, der Prostata, des Urogenitaltrakts, des lymphatischen Systems, des Magens, des Kehlkopft und/oder der Lunge einhergehen.

6. Verwendung nach Anspruch 5, wobei der Tumor aus der Gruppe Monozytenleukämie, Lungenadenokarzinom, kleinzellige Lungenkarzinome, Bauchspeicheldrüsenkrebs, Ovarialkarzinom, Glioblastome und Brustkarzinom und Kolokarzinom stammt.

7. Verwendung nach Anspruch 6, wobei die zu behandelnde Krankheit ein Tumor des Blut- und Immunsystems ist.

8. Verwendung nach Anspruch 7, wobei der Tumor aus der Gruppe der akuten myelotischen Leukämie, der chronischen myelotischen Leukämie, akuten lymphatischen Leukämie und/oder chronischen lymphatischen Leukämie stammt.

9. Verbindungen gemäß Anspruch 1 und/oder ihrer physiologisch unbedenklichen Salze zur Verwendung zur Behandlung von Tumoren wobei eine therapeutisch wirksame Menge einer Verbindung der Formel I in Kombination mit Radiotherapie und einer Verbindung aus der Gruppe 1) Ostrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

## Claims

1. Compounds selected from the group
| No. | Name and/or structure |
|---|---|
| "A1" | (4-{4-Carbamoyl-5-[3-(4-isopropylphenyl)ureido]-1H-imidazol-2-ylmethyl}phenoxy)acetic acid |
| "A2" | 4-{4-Carbamol-5-[3-(4-isopropylphenyl)ureido]-1H-imidazo)-2-ylmethyl}benzoic acid |
| | 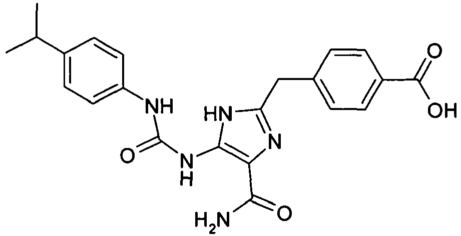 |
| "A6" | 4-{4-Carbamoyl-5-[3-(4-chlorophenyl)ureido]-1H-imidazol-2-ylmethyl)benzoic acid |
| "A7" | 4-{4-Carbamoyl-5-[3-(4-ethylphenyl)ureido]-1H-imidazol-2-ylmethyl)benzoic acid |
| "A8" | 4-{4-Carbamoyl-5-[3-(3-chloro-2-methylphenyl)ureido]-1H-imidazol-2-ylmethyl}benzoic acid |
| "A9" | 4-{5-[3-(4-Butoxyphenyl)ureido]-4-carbamoyl-1H-imidazol-2-ylmethyl)benzoic acid |
| "A10" | 5-{4-Carbamoyl-5-[3-(4-trifluoromethylphenyl)ureido]-1H-imidazol-2-ylmethyl}furan-2-carboxylic acid |
| | 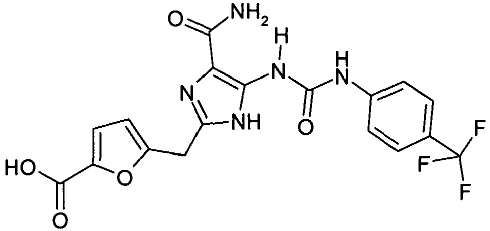 |
| "A11" | 5-{4-Carbamoyl-5-[3-(4-ethylphenyl)ureido]-1H-imidazol-2-ylmethyl}furan-2-carboxylic acid |
| "A12" | 5-{4-Carbamoyl-5-[3-(4-isopropylphenyl)ureido]-1H-imidazol-2-ylmethyl}furan-2-carboxylic acid |
| "A15" | 4-{4-Carbamoyl-5-[3-(4-trifluoromethylphenyl)ureido]-1H-imidazol-2-ylmethyl}benzoic acid |
| "A27" | 5-[4-Carbamoyl-5-(3-phenethylureido)-1H-imidazol-2-yl-methyl]furan-2-carboxylic acid |
| "A30" | 5-{4-Carbamoyl-5-[3-(4-chlorophenyl)ureido]-1H-imidazol-2-ylmethyllfuran-2-carboxylic acid |
| "A31" | 5-{4-Carbamoyl-5-[3-(2,4,6-trimethylphenyl)ureido]-1H-imidazol-2-ylmethyl}furan-2-carboxylic acid |
| "A34" | 5-{4-Carbamoyl-5-[3-(2-fluoro-5-trifluoromethylphenyl)-ureido]-1H-imidazol-2-ylmethyl}furan-2-carboxylic acid |
| "A35" | 5-{4-Carbamoyl-5-[3-(4-chloro-2-trifluoromethylphenyl)-ureido]-1H-imidazol-2-ylmethyl}furan-2-carboxylic acid |
| "A38" | (4-{4-Carbamoyl-5-[3-(4-trifluoromethylphenyl)ureido]-1H-imidazol-2-ylmethyl}phenoxy)acetic acid |
| "A39" | (4-{5-[3-(4-Butylphenyl)ureido]-4-carbamoyl-1H-imidazol-2-ylmethyl}phenoxy)acetic acid |
| "A40" | {4-[4-Carbamoyl-5-(3-p-tolylureido)-1H-imidazol-2-ylmethyl]-phenoxy}acetic acid |
| "A41" | (4-{4-Carbamoyl-5-[3-(4-chlorophenyl)ureido]-1H-imidazol-2-ylmethyl}phenoxy)acetic acid |
| "A42" | (4-{4-Carbamoyl-5-[3-(4-ethylphenyl)ureido]-1H-imidazol-2-ylmethyl}phenoxy)acetic acid |
| "A43" | (4-{4-Carbamoyl-5-[3-(4-pentylphenyl)ureido]-1H-imidazol-2-ylmethyl}phenoxy)acetic acid |
| "A49" | (4-{4-Carbamoyl-5-[3-(3-trifluoromethylphenyl)ureido]-1H-imidazol-2-yl}phenoxy)acetic acid |
| "A50" | (4-{4-Carbamoyl-5-[3-(4-trifluoromethylphenyl)ureido]-1H-imidazol-2-yl}phenoxy)acetic acid |
| "A51" | (4-{4-Carbamoyl-5-[3-(4-isopropylphenyl)ureido]-1H-imidazol-2-yl}phenoxy)acetic acid |
| "A54" | (4-{4-Carbamoyl-5-[3-(4-ethylphenyl)ureido]-1H-imidazol-2-yl}phenoxy)acetic acid |
| "A55" | (4-{4-Carbamoyl-5-[3-(2-fluoro-5-trifluoromethylphenyl)-ureido]-1H-imidazol-2-yl}phenoxy)acetic acid |
| "A56" | (4-{4-Carbamoyl-5-[3-(4-trifluoromethylphenyl)ureido]-1H-imidazol-2-ylmethoxy}phenyl)acetic acid |
| "A57" | (4-{4-Carbamoyl-5-[3-(4-isopropylphenyl)ureido]-1H-imidazol-2-ylmethoxy}phenyl)acetic acid |
| "A60" | (4-{4-Carbamoyl-5-[3-(2,4-dimethylphenyl)ureido]-1H-imidazol-2-ylmethoxy}phenyl)acetic acid |
| "A61" | (4-{4-Carbamoyl-5-[3-(4-ethylphenyl)ureido]-1H-imidazol-2-ylmethoxy}phenyl)acetic acid |
| "A62" | (4-{4-Carbamoyl-5-[3-(4-chloro-2-methylphenyl)ureido]-1H-imidazol-2-ylmethoxy}phenyl)acetic acid |
| "A66" | 5-{4-Carbamoyl-5-[3-(4-triftuoromethylphenyl)ureido]-1H-imidazol-2-ylmethyl}thiophene-2-carboxylic acid |
| "A68" | 5-{4-Carbamoyl-5-[3-(4-isopropylphenyl)ureido]-1H-imidazol-2-ylmethyl}thiophene-2-carboxylic acid |
| "A70" | 5-{4-Carbamoyl-5-[3-(4-chlorophenyl)ureido-1H-imidazol-2-ylmethyl}thiophene-2-carboxylic acid |
| "A71" | 5-{4-Carbamoyl-5-[3-(2,4-dimethylphenyl)ureido]-1H-imidazol-2-ylmethyl}thiophene-2-carboxylic acid |
| "A72" | 5-{4-Carbamoyl-5-[3-(4-ethylphenyl)ureido]-1H-imidazol-2-ylmethyl}thiophene-2-carboxylic acid |
| "A74" | 5-{4-Carbamoyl-5-[3-(4-chloro-2-methylphenyl)ureido]-1H-imidazol-2-ylmethyl}thiophene-2-carboxylic acid |
| "A84" | 5-[3-(4-Butylphenyl)ureido]-2-[5-(methylphenylcarbamoyl)-furan-2-ylmethyl]-1H-imidazole-4-carboxamide |
| | 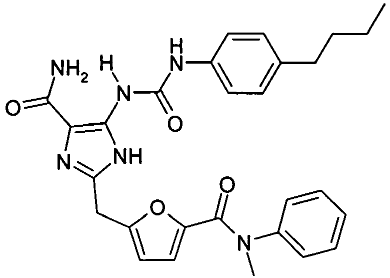 |
| "A85" | 5-[3-(4-Butylphenyl)ureido]-2-[5-(4-methylpiperazine-1-carbonyl)furan-2-ylmethyl]-1H-imidazole-4-carboxamide |
| "A91" | 5-[3-(4-Butylphenyl)ureido]-2-{5-[4-(2-hydroxyethyl)-piperidine-1-carbonyl]furan-2-ylmethyl}-1H-imidazole-4-carboxamide |
| "A93" | 4-{4-Carbamoyl-5-[3-(4-trifluoromethoxyphenyl)ureido]-1H-imidazol-2-ylmethyl}benzoic acid |
| "A94" | 5-[3-(4-Butylphenyl)ureido]-2-[4-(morpholine-4-carbonyl)-benzyl]-1H-imidazole-4-carboxamide |
| "A97" | from "A96" by reduction using Fe powder/NH₄Cl: 2-(4-aminobenzyl)-5-[3-(4-trifluoromethylphenyl)ureido]-1H-imidazole-4-carboxamide |
| "A102" | 4-[4-Carbamoyl-5-(3-thiophen-2-ylureido)-1H-imidazol-2-yl-methyl]benzoic acid |
| | |
| | |
|---|---|
| "B2" | 2-[4-(3,5-Dimethylpyrazole-1-carbonyl)benzyl]-5-[3-(4-trifluoromethylphenyl)ureido]-1H-imidazole-4-carboxamide, |
| "B4" | 5-[3-(4-Isopropylphenyl)ureido]-2-(4-methoxybenzyl)-1H-imidazole-4-carboxamide, |
| "B7" | 3-{3-[5-Carbamoyl-2-(4-methoxybenzyl)-3H-imidazol-4-yl]-ureido}benzoic acid ester, |
| "B10" | 5-[3-(4-Isopropylphenyl)ureido]-2-f4-[2-(4-methylpiperazin-1-yl)-2-oxoethoxy]benzyl}-1H-imidazole-4-carboxamide, |
| "B11" | {4-[5-(3-Benzo-1,3-dioxol-5-ylureido)-4-carbamoyl-1H-imidazol-2-ylmethyl]phenoxylacetic acid, |
| "B12" | 2-(4-Methanesulfonylbenzyl)-5-[3-(4-trifluoromethylphenyl)-ureido]-1H-imidazole-4-carboxamide, |
| "B19" | (4-{4-Carbamoyl-5-[3-(2,7a-dihydrobenzofuran-5-yl)ureido]-1 H-imidazol-2-ylmethyl}phenoxy)acetic acid, |
| "B20" | {4-[5-(3-Benzo[b]thiophen-5-ylureido)-4-carbamoyl-1H-imidazol-2-ylmethyl]phenoxy}acetic acid, |
| "B25" | (4-{4-Carbamoyl-5-[3-(2,3-dimethylphenyl)ureido]-1H-imidazol-2-ylmethyl}phenoxy)acetic acid, |
| "B28" | 2-[4-(4-Pyridin-2-ylmethylpiperazine-1-carbonyl)benzyl]-5-[3-(4-trifluoromethylphenyl)ureido]-1H-imidazole-4-carboxamide, |
| "B30" | 2-{4-[(Pyridin-2-ylmethyl)carbamoyl]benzyl}-5-[3-(4-trifluoromethylphenyl)ureido]-1H-imidazole-4-carboxamide, |
| "B31" | 4-{5-[3-(4-tert-Butylphenyl)ureido]-4-carbamoyl-1H-imidazol-2-ylmethyl}benzoic acid, |
| "B33" | 4-{4-Carbamoyl-5-[3-(3-chloro-4-methylphenyl)ureido]-1H-imidazol-2-ylmethyl}benzoic acid, |
| "B34" | 2-[4-(2-Pyridin-3-ylacetylamino)benzyl]-5-[3-(4-trifluoromethylphenyl)ureido]-1H-imidazole-4-carboxamide, |
| "B35" | 2-[4-(2-1H-)midazol-4-ylacetylamino)benzyl]-5-[3-(4-trifluoromethylphenyl)ureido]-1H-imidazole-4-carboxamide, |
| "B36" | Ethyl (4-{4-carbamoyl-5-[3-(4-isopropylphenyl)ureido]-1H-imidazol-2-ylmethyl}phenoxy)acetate, |
| "B44" | (4-{4-Carbamoyl-5-[3-(4-isopropyl-3-methylphenyl)ureido]-1 H-imidazol-2-ylmethyl}phenoxy)acetic acid, |
| "B51" | (4-{4-Carbamoyl-5-[3-(3,4-dichlorophenyl)ureido]-1H-imidazol-2-ylmethyl}phenoxy)acetic acid, |
| "B52" | (4-{4-Carbamoyl-5-[2-(4-chlorophenyl)acetylamino]-1H-imidazol-2-ylmethyl}phenoxy)acetic acid, |
| "B53" | 2-(4-Hydroxycarbamoylbenzyl)-5-[3-(4-trifluoromethyl-phenyl)ureido]-1H-imidazole-4-carboxamide, |
| "B54" | 2-[4-(Hydroxymethylcarbamoyl)benzyl]-5-[3-(4-trifluoromethylphenyl)ureido]-1H-imidazole-4-carboxamide, |
| "B55" | (4-{4-Carbamoyl-5-[3-(4-nitrophenyl)ureido]-1H-imidazol-2-ylmethyl}phenoxy)acetic acid |
| "B56" | (4-{4-Carbamoyl-5-[3-(4-cyanophenyl)ureido]-1H-imidazol-2-ylmethyl}phenoxy)acetic acid, |
| "B57" | (4-{4-Carbamoyl-5-[3-(2,4-dichlorophenyl)ureido]-1H-imidazol-2-ylmethyl}phenoxy)acetic acid, |
| "B62" | 2-[2-(1H-Benzotriazol-5-ylcarbamoyl)ethyl]-5-[3-(4-trifluoromethylphenyl)ureido]-1H-imidazole-4-carboxamide, |
| "B64" | (4-{4-Carbamoyl-5-[3-(4-trifluoromethylsulfanylphenyl)-ureido]-1H-imidazol-2-ylmethyl}phenoxy)acetic acid, |
| "B65" | 2-[3-(1H-Benzotriazol-5-ylcarbamoyl)propyl]-5-[3-(4-chlorophenyl)ureido]-1H-imidazole-4-carboxylic acid, |
| "B66" | 5-[3-(4-Chlorophenyl)ureido]-2-[3-(2-trifluoromethyl-1H-benzimidazol-5-ylcarbamoyl)propyl]-1H-imidazole-4-carboxylic acid, |
| "B67" | 5-[3-(4-Chlorophenyl)ureido]-2-[3-(1H-indazol-5-yl-carbamoyl)propyl]-1H-imidazole-4-carboxylic acid, |
| "B70" | 2-[3-(1H-Benzotriazol-5-ylcarbamoyl)propyl]-5-[3-(4-trifluoromethylphenyl)ureido]-1H-imidazole-4-carboxylic acid, |
| "B71" | 2-[3-(2-Oxo-2,3-dihydrobenzoxazol-6-ylcarbamoyl)propyl]-5-[3-(4-trifluoromethylphenyl)ureido]-1H-imidazole-4-carboxylic acid, |
| "B72" | 2-[3-(1H-Indazol-5-ylcarbamoyl)propyl]-5-[3-(4-trifluoromethylphenyl)ureido]-1H-imidazole-4-carboxylic acid, |
| "B73" | 2-[3-(2-Trifluoromethyl-1 H-benzimidazol-5-ylcarbamoyl)-propyl]-5-[3-(4-trifluoromethylphenyl)ureido]-1H-imidazole-4-carboxylic acid, |
| "B74" | 2-[2-(2-Oxo-2,3-dihydrobenzoxazol-6-ylcarbamoyl)ethyl]-5-[3-(4-trifluoromethylphenyl)ureido]-1H-imidazole-4-carboxylic acid, |
| "B76" | 2-(3-{4-Carbamoyl-5-[3-(4-chlorophenyl)ureido]-1H-imidazol-2-yl)phenoxy)-2-methylpropionic acid, |
| "B77" | 5-[3-(4-Chlorophenyl)ureido]-2-(1,3-dioxo-1,3-dihydroisoindol-2-ylmethyl)-1H-imidazole-4-carboxamide, |
| "B78" | 5-[3-(4-Chlorophenyl)ureido]-2-(1,3-dioxo-1,3-dihydroisoindol-2-ylmethyl)-1H-imidazole-4-carboxamide, |
| "B79" | 5-[3-(4-Chlorophenyl)ureido]-2-[2-(2-oxo-2,3-dihydrobenzoxazol-6-ylcarbamoyl)ethyl]-1H-imidazole-4-carboxamide or |
| "B80" | 2-[2-(1H-Benzotriazol-5-ylcarbamoyl)ethyl]-5-[3-(4-chlorophenyl)ureido]-1H-imidazole-4-carboxamide, |
and pharmaceutically usable tautomers, salts and stereoisomers thereof, including mixtures thereof in all ratios.

2. Medicaments comprising at least one compound according to Claim 1 and/or pharmaceutically usable salts, solvates, tautomers and stereoisomers thereof, including mixtures thereof in all ratios, and optionally excipients and/or adjuvants.

3. Use of compounds according to Claim 1 and pharmaceutically usable salts, solvates, tautomers and stereoisomers thereof, including mixtures thereof in all ratios, for the preparation of a medicament for the treatment of diseases in which the inhibition, regulation and/or modulation of phosphodiesterase or lysophospholipase autotaxin plays a role.

4. Use of compounds according to Claim 1 for the preparation of a medicament for the treatment and prophylaxis of cancer diseases.

5. Use according to Claim 4, where the cancer diseases are associated with a tumour from the group of tumours of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx and/or of the lung.

6. Use according to Claim 5, where the tumour originates from the group monocytic leukaemia, lung adenocarcinoma, small-cell lung carcinomas, pancreatic cancer, ovarian carcinoma, glioblastomas and breast carcinoma and colon carcinoma.

7. Use according to Claim 6, where the disease to be treated is a tumour of the blood and immune system.

8. Use according to Claim 7, where the tumour originates from the group of acute myeloid leukaemia, chronic myeloid leukaemia, acute lymphatic leukaemia and/or chronic lymphatic leukaemia.

9. Compounds according to Claim 1 and/or physiologically acceptable salts thereof for use for the treatment of tumours, where a therapeutically effective amount of a compound of the formula I is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

## Revendications

1. Composés choisis dans le groupe constitué par
| n° | Nom et/ou structure |
|---|---|
| « A1 » | Acide (4-{4-carbamoyl-5-[3-(4-isopropylphényl)uréido]-1H-imidazol-2-ylméthyl}phénoxy)acétique |
| « A2 » | Acide 4-{4-carbamoyl-5-[3-(4-isopropylphényl)uréido]-1H-imidazol-2-ylméthyl}benzoïque |
| | 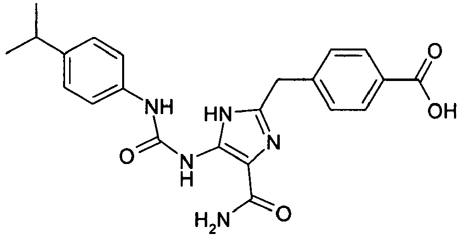 |
| « A6 » | Acide 4-{4-carbamoyl-5-[3-(4-chtorophényl)uréido]-1H-imidazol-2-ylméthyl}benzoïque |
| « A7 » | Acide 4-{4-carbamoyl-5-[3-(4-éthylphényl)uréido]-1H-imidazol-2-ylméthyl}benzoïque |
| « A8 » | Acide 4-{4-carbamoyl-5-[3-(3-chloro-2-méthylphényl)-uréido]-1H-imidazol-2-ylméthyl}benzoïque |
| « A9 » | Acide 4-{5-[3-(4-butoxyphényl)uréido]-4-carbamoyl-1H-imidazol-2-ylméthyl}benzoïque |
| « A10 » | Acide 5-{4-carbamoyl-5-[3-(4-trifluorométhylphényl)uréido]-1H-imidazol-2-ylméthyl}furan-2-carboxylique |
| | 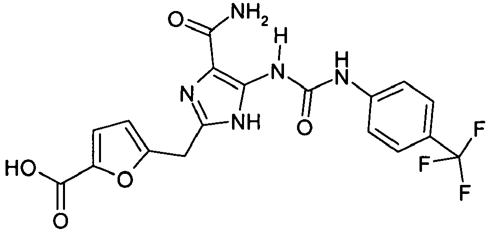 |
| « A11 » | Acide 5-{4-carbamoyl-5-[3-(4-éthylphényl)uréidol-1H-imidazol-2-ylméthyl}furan-2-carboxylique |
| « A12 » | Acide 5-{4-carbamoyl-5-[3-(4-isopropylphényl)uréido]-1H-imidazol-2-ylméthyl}furan-2-carboxylique |
| « A15 » | Acide 4-{4-carbamoyl-5-[3-(4-trifluorométhylphényl)uréido]-1H-imidazol-2-ylméthyl}benzoïque |
| « A27 » | Acide 5-[4-carbamoyl-5-(3-phénéthyluréido)-1H-imidazol-2-ylméthyl]furan-2-carboxyliq ue |
| « A30 » | Acide 5-{4-carbamoyl-5-[3-(4-chlorophényl)uréido]-1H-imidazol-2-ylméthyl}furan-2-carboxylique |
| « A31 » | Acide 5-{4-carbamoyl-5-[3-(2,4,6-triméthylphényl)uréido]-1H-imidazol-2-ylméthyl}furan-2-carboxylique |
| « A34 » | Acide 5-{4-carbamoyl-5-[3-(2-fluoro-5-trifluorométhylphényl)uréido]-1H-imidazol-2-ylméthyl}furan-2-carboxylique |
| « A35 » | Acide 5-{4-carbamoyl-5-[3-(4-chloro-2-trifluorométhyl-phényl)uréido]-1H-imidazol-2-ylméthyl}furan-2-carboxylique |
| « A38 » | Acide (4-{4-carbamoyl-5-[3-(4-trifluorométhylphényl)-uréido]-1H-imidazol-2-ylméthyl}phénoxy)acétique |
| « A39 » | Acide (4-{5-[3-(4-butylphényl)uréido]-4-carbamoyl-1H-imidazol-2-ylméthyl}phénoxy)acétique |
| « A40 » | Acide {4-[4-carbamoyl-5-(3-p-tolyluréido)-1H-imidazol-2-yl-méthyl]phénoxy}acétique |
| « A41 » | Acide (4-{4-carbamoyl-5-[3-(4-chlorophényl)uréido]-1H-imidazol-2-ylméthyl}phénoxy)acétique |
| « A42 » | Acide (4-{4-carbamoyl-5-[3-(4-éthylphényl)uréido]-1H-imidazol-2-ylméthyl}phénoxy)acétique |
| « A43 » | Acide (4-{4-carbamoyl-5-[3-(4-pentylphényl)uréido]-1H-imidazol-2-ylméthyl}phénoxy)acétique |
| « A49 » | Acide (4-{4-carbamoyl-5-[3-(3-trifluorométhylphényl)-uréido]-1H-imidazol-2-yl}phénoxy)acétique |
| « A50 » | Acide (4-{4-carbamoyl-5-[3-(4-trifluorométhylphényl)-uréido]-1H-imidazol-2-yl}phénoxy)acétique |
| « A51 » | Acide (4-{4-carbamoyl-5-[3-(4-isopropylphényl)uréido]-1H-imidazol-2-yl}phénoxy)acétique |
| « A54 » | Acide (4-{4-carbamoyl-5-[3-(4-éthylphényl)uréido]-1H-imidazol-2-yl}phénoxy)acétique |
| « A55 » | Acide (4-{4-carbamoyl-5-[3-(2-fluoro-5-trifluorométhyl-phényl)uréido]-1H-imidazol-2-yl}phénoxy)acétique |
| « A56 » | Acide (4-{4-carbamoyl-5-[3-(4-trifluorométhylphényl)-uréido]-1H-imidazol-2-ylméthoxy}phényl)acétique |
| « A57 » | Acide (4-{4-carbamoyl-5-[3-(4-isopropylphényl)uréido]-1H-imidazol-2-ylméthoxy}phényl)acétique |
| « A60 » | Acide (4-{4-carbamoyl-5-[3-(2,4-diméthylphényl)uréido]-1H-imidazol-2-ylméthoxy}phényl)acétique |
| « A61 » | Acide (4-{4-carbamoyl-5-[3-(4-éthylphényl)uréido]-1H-imidazol-2-ylméthoxy}phényl)acétique |
| « A62 » | Acide (4-{4-carbamoyl-5-[3-(4-chloro-2-méthylphényl)-uréido]-1H-imidazol-2-ylméthoxy}phényl)acétique |
| « A66 » | Acide 5-{4-carbamoyl-5-[3-(4-trifluorométhylphényl)uréido]-1H-imidazol-2-ylméthyl}thiophène-2-carboxylique |
| « A68 » | Acide 5-{4-carbamoyl-5-[3-(4-isopropylphényl)uréido]-1H-imidazol-2-ylméthyl}thiophène-2-carboxylique |
| « A70 » | Acide 5-{4-carbamoyl-5-[3-(4-chlorophényl)uréido]-1H-imidazol-2-ylméthyl}thiophène-2-carboxylique |
| « A71 » | Acide 5-{4-carbamoyl-5-[3-(2,4-diméthylphényl)uréido]-1H-imidazol-2-ylméthyl}thiophène-2-carboxylique |
| « A72 » | Acide 5-{4-carbamoyl-5-[3-(4-éthylphényl)uréidol-1H-imidazol-2-ylméthyl}thiophène-2-carboxylique |
| « A74 » | Acide 5-{4-carbamoyl-5-[3-(4-chloro-2-méthylphényl)-uréido]-1H-imidazol-2-ylméthyl}thiophène-2-carboxylique |
| « A84 » | 5-[3-(4-Butylphényl)uréido]-2-[5-(méthylphénylcarbamoyl)-furan-2-ylméthyl]-1H-imidazole-4-carboxamide |
| | 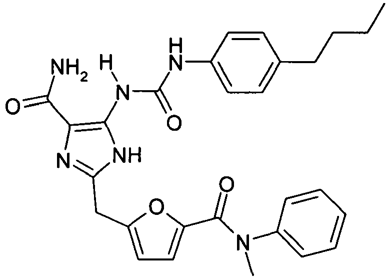 |
| « A85 » | 5-[3-(4-Butylphényl)uréido]-2-[5-(4-méthylpipérazine-1-carbonyl)furan-2-ylméthyl]-1H-imidazole-4-carboxamide |
| « A91 » | 5-[3-(4-Butylphényl)uréido]-2-{5-[4-(2-hydroxyéthyl)-pipéridine-1-carbonyl]furan-2-ylméthyl}-1H-imidazole-4-carboxamide |
| « A93 » | Acide 4-{4-carbamoyl-5-[3-(4-trifluorométhoxyphényl)-uréido]-1H-imidazol-2-ylméthyl}benzoïque |
| « A94 » | 5-[3-(4-Butylphényl)uréido]-2-[4-(morpholine-4-carbonyl)benzyl]-1H-imidazole-4-carboxamide |
| « A97 » | à partir de « A96 » par réduction à l'aide de poudre de Fe/NH₄Cl : 2-(4-aminobenzyl)-5-[3-(4-trifluorométhylphényl)uréido]-1H-imidazole-4-carboxamide |
| « A102 » | Acide 4-[4-carbamoyl-5-(3-thiophén-2-yluréido)-1H-imidazol-2-ylméthyl]benzoïque |
| | |
|---|---|
| « B2 » | 2-[4-(3,5-Diméthylpyrazole-1-carbonyl)benzyl]-5-[3-(4-trifluorométhylphényl)uréido]-1H-imidazole-4-carboxamide, |
| « B4 » | 5-[3-(4-Isopropylphényl)uréido]-2-(4-méthoxybenzyl)-1H-imidazole-4-carboxamide, |
| « B7 » | Ester de l'acide 3-(3-[5-carbamoyl-2-(4-méthoxybenzyl)-3H-imidazol-4-yl]uréido}benzoïque, |
| « B10 » | 5-[3-(4-Isopropylphényl)uréido]-2-{4-[2-(4-méthylpipérazin-1-yl)-2-oxoéthoxy]benzyl}-1H-imidazole-4-carboxamide, |
| « B11 » | Acide {4-[5-(3-benzo-1,3-dioxol-5-yluréido)-4-carbamoyl-1H-imidazol-2-ylméthyl]phénoxy}acétique, |
| « B12 » | 2-(4-Méthanesulfonylbenzyl)-5-[3-(4-trifluorométhylphényl)-uréido]-1H-imidazole-4-carboxamide, |
| « B19 » | Acide (4-{4-carbamoyl-5-[3-(2,7a-dihydrobenzofuran-5-yl)-uréido]-1H-imidazol-2-ylméthyl}phénoxy)acétique, |
| « B20 » | Acide {4-[5-(3-benzo[b]thiophén-5-yluréido)-4-carbamoyl-1H-imidazol-2-ylméthyl]phénoxy}acétique, |
| « B25 » | Acide (4-{4-carbamoyl-5-[3-(2,3-diméthylphényl)uréido]-1H-imidazol-2-ylméthyl}phénoxy)acétique, |
| « B28 » | 2-[4-(4-Pyridin-2-ylméthylpipérazine-1-carbonyl)benzyl]-5-[3-(4-trifluorométhylphényl)uréido]-1H-imidazole-4-carboxamide, |
| « B30 » | 2-{4-[(Pyridin-2-ylméthyl)carbamoyl]benzyl}5-[3-(4-trifluorométhylphényl)uréido]-1H-imidazole-4-carboxamide, |
| « B31 » | Acide 4-{5-[3-(4-tertio-butylphényl)uréido]-4-carbamoyl-1H-imidazol-2-ylméthyl}benzoïque, |
| « B33 » | Acide 4-{4-carbamoyl-5-[3-(3-chloro-4-méthylphényl)uréido]-1H-imidazol-2-ylméthyl}benzoïque, |
| « B34 » | 2-[4-(2-Pyridin-3-ylacétylamino)benzyl]-5-[3-(4-trifluorométhylphényl)uréido]-1H-imidazole-4-carboxamide, |
| « B35 » | 2-[4-(2-1H-Imidazol-4-ylacétylamino)benzyl]-5-[3-(4-trifluorométhylphényl)uréido]-1H-imidazole-4-carboxamide, |
| « B36 » | (4-{4-Carbamoyl-5-[3-(4-isopropylphényl)uréido]-1H-imidazol-2-ylméthyl}phénoxy)acétate d'éthyle, |
| « B44 » | Acide (4-{4-carbamoyl-5-[3-(4-isopropyl-3-méthylphényl)-uréido]-1H-imidazol-2-ylméthyl}phénoxy)acétique, |
| « B51 » | Acide (4-{4-carbamoyl-5-[3-(3,4-dichlorophényl)uréidol-1H-imidazol-2-ylméthyl}phénoxy)acétique, |
| « B52 » | Acide (4-{4-carbamoyl-5-[2-(4-chlorophényl)acétylamino]-1H-imidazol-2-ylméthyl}phénoxy)acétique, |
| « B53 » | 2-(4-Hydroxycarbamoylbenzyl)-5-[3-(4-trifluorométhyl-phényl)uréido]-1H-imidazole-4-carboxamide, |
| « B54 » | 2-[4-(Hydroxyméthylcarbamoyl)benzyl]-5-[3-(4-trifluorométhylphényl)uréido]-1H-imidazole-4-carboxamide, |
| « B55 » | Acide (4-{4-carbamoyl-5-[3-(4-nitrophényl)uréido]-1H-imidazol-2-ylméthyl}phénoxy)acétique |
| « B56 » | Acide (4-{4-carbamoyl-5-[3-(4-cyanophényl)uréido]-1H-imidazol-2-ylméthyl}phénoxy)acétique, |
| « B57 » | Acide (4-{4-carbamoyl-5-[3-(2,4-dichlorophényl)uréido]-1H-imidazol-2-ylméthyl}phénoxy)acétique, |
| « B62 » | 2-[2-(1H-Benzotriazol-5-ylcarbamoyl)éthyl]-5-[3-(4-trifluorométhylphényl)uréido]-1H-imidazole-4-carboxamide, |
| « B64 » | Acide (4-{4-carbamoyl-5-[3-(4-trifluorométhylsulfanyl-phényl)uréido]-1H-imidazol-2-ylméthyl}phénoxy)acétique, |
| « B65 » | Acide 2-[3-(1H-benzotriazol-5-ylcarbamoyl)propyl]-5-[3-(4-chlorophényl)uréido]-1H-imidazole-4-carboxylique, |
| « B66 » | Acide 5-[3-(4-chlorophényl)uréido]-2-[3-(2-trifluorométhyl-1 H-benzimidazol-5-ylcarbamoyl)propyl]-1H-imidazole-4-carboxylique, |
| « B67 » | Acide 5-[3-(4-chlorophényl)uréido]-2-[3-(1H-indazol-5-yl-carbamoyl)propyl]-1H-imidazole-4-carboxylique, |
| « B70 » | Acide 2-[3-(1H-benzotriazol-5-ylcarbamoyl)propyl]-5-[3-(4-trifluorométhylphényl)uréido]-1H-imidazole-4-carboxylique, |
| « B71 » | Acide 2-[3-(2-oxo-2,3-dihydrobenzoxazol-6-ylcarbamoyl)-propyl]-5-[3-(4-trifluorométhylphényl)uréido]-1H-imidazole-4-carboxylique, |
| « B72 » | Acide 2-[3-(1 H-indazol-5-ylcarbamoyl)propyl]-5-[3-(4-trifluorométhylphényl)uréido]-1H-imidazole-4-carboxylique, |
| « B73 » | Acide 2-[3-(2-trifluorométhyl-1H-benzimidazol-5-yl-carbamoyl)propyl]-5-[3-(4-trifluorométhylphényl)uréido]-1H-imidazole-4-carboxylique, |
| « B74 » | Acide 2-[2-(2-oxo-2,3-dihydrobenzoxazol-6-ylcarbamoyl)-éthyl]-5-[3-(4-trifluorométhylphényl)uréido]-1H-imidazole-4-carboxylique, |
| « B76 » | Acide 2-(3-{4-carbamoyl-5-[3-(4-chlorophényl)uréido]-1H-imidazol-2-yl}phénoxy)-2-méthylpropionique, |
| « B77 » | 5-[3-(4-Chlorophényl)uréido]-2-(1,3-dioxo-1,3-dihydroisoindol-2-ylméthyl)-1H-imidazole-4-carboxamide, |
| « B78 » | 5-[3-(4-Chlorophényl)uréido]-2-(1,3-dioxo-1,3-dihydroisoindol-2-ylméthyl)-1H-imidazole-4-carboxamide, |
| « B79 » | 5-[3-(4-Chlorophényl)uréido]-2-[2-(2-oxo-2,3-dihydrobenzoxazol-6-ylcarbamoyl)éthyl]-1H-imidazole-4-carboxamide ou |
| « B80 » | 2-[2-(1H-Benzotriazol-5-ylcarbamoyl)éthyl]-5-[3-(4-chlorophényl)uréido]-1H-imidazole-4-carboxamide, |
ainsi que les tautomères, sels et stéréoisomères pharmaceutiquement utilisables de ceux-ci, y compris des mélanges de ceux-ci en toutes proportions.

2. Médicaments comprenant au moins un composé selon la revendication 1 et/ou des sels, solvates, tautomères et stéréoisomères pharmaceutiquement utilisables de celui-ci, y compris des mélanges de ceux-ci en toutes proportions, et éventuellement des excipients et/ou des adjuvants.

3. Utilisation de composés selon la revendication 1 et de sels, solvates, tautomères et stéréoisomères pharmaceutiquement utilisables de ceux-ci, y compris des mélanges de ceux-ci en toutes proportions, pour la préparation d'un médicament destiné au traitement de maladies dans lesquelles l'inhibition, la régulation et/ou la modulation de la phosphodiestérase ou de la lysophospholipase autotaxine joue un rôle.

4. Utilisation de composés selon la revendication 1, pour la préparation d'un médicament destiné au traitement et à la prophylaxie de maladies cancéreuses.

5. Utilisation selon la revendication 4, dans laquelle les maladies cancéreuses sont associées à une tumeur issue du groupe des tumeurs de l'épithélium squameux, de la vessie, de l'estomac, du rein, de la tête et du cou, de l'oesaphage, du col de l'utérus, de la thyroïde, des intestins, du foie, du cerveau, de la prostate, du tractus urogénital, du système lymphatique, de l'estomac, du larynx et/ou du poumon.

6. Utilisation selon la revendication 5, dans laquelle la tumeur est issue du groupe de la leucémie monocytique, de l'adénocarcinome du poumon, des carcinomes du poumon à petites cellules, du cancer du pancréas, du carcinome de l'ovaire, des glioblastomes et du carcinome du sein et du carcinome du côlon.

7. Utilisation selon la revendication 6, dans laquelle la maladie à traiter est une tumeur du sang et du système immunitaire.

8. Utilisation selon la revendication 7, dans laquelle la tumeur est issue du groupe de la leucémie myéloïde aiguë, la leucémie myéloïde chronique, la leucémie lymphatique aiguë et/ou la leucémie lymphatique chronique.

9. Composés selon la revendication 1 et/ou des sels physiologiquement acceptables de ceux-ci, pour une utilisation dans le traitement de tumeurs, où une quantité thérapeutiquement efficace d'un composé de formule I est administrée en association avec une radiothérapie et un composé issu du groupe : 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.
